# EUROPEAN PATENT SPECIFICATION

(11) **EP 2 118 664 B1**
(45) Date of publication and mention of the grant of the patent: **07.03.2012**
(21) Application number: 08705059.7
(22) Date of filing: 14.01.2008
(51) Int. Cl.: G01N 33/68

(54) **PEPTIDE MARKERS FOR DIAGNOSIS OF PREECLAMPSIA**
PEPTIDMARKER ZUR DIAGNOSE VON PRÄEKLAMPSIE
PEPTIDES MARQUEURS POUR LE DIAGNOSTIC DE L'ECLAMPSISME

(30) Priority: 12.01.2007 WO PCT/NL2007/050014
(43) Date of publication of application: 18.11.2009
(73) Proprietor: Erasmus University Medical Center Rotterdam, 3015 GE Rotterdam (NL)
(72) Inventor: LUIDER, Theo, Marten, NL-3069 XK Rotterdam (NL); SILLEVIS SMITT, Petrus, Abraham, Elisa, NL-3016 DL Rotterdam (NL); STEEGERS, Eric Adrianus Petrus, NL-3015 GE Rotterdam (NL)
(74) Representative: Hatzmann, Martin
(86) International application number: PCT/NL2008/000021
(87) International publication number: WO 2008/085036

(56) References cited:
- SCHEEPERS ET AL: "Advances in the discovery of low-abundance proteins" TRAC, TRENDS IN ANALYTICAL CHEMISTRY, ELSEVIER, AMSTERDAM, NL, vol. 25, no. 9, October 2006 (2006-10), pages 841-847, XP005681788 ISSN: 0165-9936
- DE GROOT CHRISTIANNE J M ET AL: "Specific peptides identified by mass spectrometry in placental tissue from pregnancies complicated by early onset preeclampsia attained by laser capture dissection" PROTEOMICS CLINICAL APPLICATIONS, vol. 1, no. 3, March 2007 (2007-03), pages 325-335, XP002477511 ISSN: 1862-8346
- BISCHOFF ET AL: "Biomarker discovery by mass spectrometry symposium, May 18-19, 2006" JOURNAL OF CHROMATOGRAPHY B: BIOMEDICAL SCIENCES & APPLICATIONS, ELSEVIER, AMSTERDAM, NL, vol. 847, no. 1, 18 May 2006 (2006-05-18), - 18 May 2006 (2006-05-18) pages 1-2, XP005881030 09-02-2007 ISSN: 1570-0232
- GUPTA ANURAG KUMAR ET AL: "Analysis of plasma elastase levels in early and late onset preeclampsia." ARCHIVES OF GYNECOLOGY AND OBSTETRICS JAN 2006, vol. 273, no. 4, January 2006 (2006-01), pages 239-242, XP002477512 ISSN: 0932-0067
- DE GROOT CHRISTIANNE J M ET AL: "Peptide patterns of laser dissected human trophoblasts analyzed by matrix-assisted laser desorption/ionisation-time of flight mass spectrometry." PROTEOMICS FEB 2005, vol. 5, no. 2, February 2005 (2005-02), pages 597-607, XP002477513 ISSN: 1615-9853

## Description

### FIELD OF THE INVENTION

The present invention is in the field of disease diagnostics. The invention provides the use of calcyclin as a marker for the diagnosis, or (therapeutic) monitoring of early-onset preeclampsia.

### BACKGROUND OF THE INVENTION

Preeclampsia is a pregnancy specific syndrome that is diagnosed by the new appearance of increased blood pressure and proteinuria. It is a leading cause of maternal mortality in developed countries and increases perinatal mortality up to five-fold. Since its etiology is largly unknown, a panoply of pathophysiological abnormalities are described. However, it is evident that abnormal placentation plays an important role. In normal pregnancy spiral arteries undergo striking remodeling. They change from typical muscular arteries to flaccid tubes with no muscularis or elastic lamina with a diameter at least four times greater than that of non-pregnant vessels. Shortly after the invasion of trophoblasts into the superficial endometrium, the maternal erythrocytes can be observed within the precursors of the placental intervillous space. In women with preeclampsia endovascular remodelling and invasion of the spiral arteries is less prominent which is assumed to result in overwhelming placental oxidative stress and pregnancy failure. However, there are at present no diagnostic markers for detecting early onset preeclampsia in pregnant women.

Rapid and major developments in proteomic technology and methodology over the last decade have opened a new stage in the identification of proteins. Matrix-assisted laser desorption/ionization time-of-flight mass spectrometry (MALDI-TOFMS) recently became available as a flexible tool in the search for disease markers. Moreover, the recently introduced technique of matrix-assisted laser desorption/ionization Fourier transform mass spectrometry (MALDI-FTMS) provides a powerful technique for accurate peptide mass measurements. This technique has successfully been used for studies in protein interactions and post-translational modifications of proteins.

It is known that the protein calcyclin is present in mature placenta tissue after birth at the end of the pregnancy (40 weeks gestation) in late onset preeclampsia. There is, however, a need to assess the risk or the presence of early onset preeclampsia, which by definition is at a much earlier stage in the pregnancy, and which requires in vivo biopsies. Kumar et al. (2006. Arch Gynecol Obstet., Vol 273, 239-242) discloses elastase as a marker of early onset pre-eclampsia in plasma. Therefore, there is a need for biomarkers, in particular protein or peptide markers that can indicate the risk of early onset preeclampsia or the presence of early onset preeclampsia with great precision at an early stage, preferably between 10 and 34 weeks.

### SUMMARY OF THE INVENTION

The invention is defined by the claims. The present inventors set out to analyze, identify, and verify specific proteins of villous trophoblast and villous stroma in small numbers of microdissected cells (approximately 125 cells) from seven placentas of women with pregnancies complicated by preeclampsia (cases) and seven uncomplicated pregnancies (controls). Tryptic peptide profiling by MALDI-TOF MS was used for comparison and identification of significantly expressed peptides. The data were analyzed by ClinProTools (Bruker Daltonics) and by principal component analysis. Subsequently, a subset of placental tissues were homogenized and separated on a NanoLC system to obtain sequencing information (MS/MS spectra). Specific peptide patterns were identified in the different cell types: villous stromal and trophoblast cells and differences in these cells of placentas from women with pregnancies complicated by early compared to late onset preeclampsia (<34 and >34 wk gestation, respectively) and controls. Principal component analysis revealed significant differences between the groups. The comparison with placental tissue after preterm delivery with unknown cause revealed that placental peptide patterns in early onset preeclampsia could not be explained by preterm delivery per se. Subsequently, specific, discriminating proteins for early onset preeclampsia compared to controls were identified including calcyclin, surfeit locus protein, and choriomammotropin A precursor. The expression of calcyclin was verified in early onset preeclamptic placental sections by immunohistochemistry. These data suggest that in early onset preeclampsia trophoblastic choriomammotropin regulation is abnormal, possibly through abnormal calcyclin expression and regulation. The present inventors have thus discovered a method to detect early onset preeclampsia, which method involves testing the presence of calcyclin. Further, surfeit locus protein, and/or choriomammotropin A in chorionic villi, preferably trophoblasts may be tested in addition to calcyclin.

### SHORT DESCRIPTION OF THE DRAWINGS

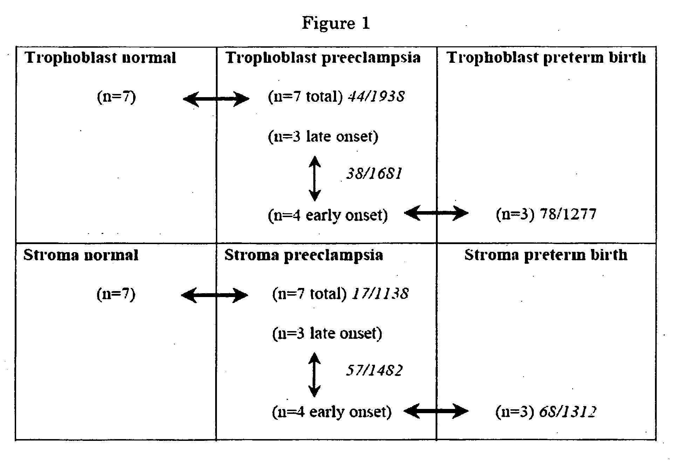

Fig. 1 represents a chart that displays the various comparisons made between trophoblast versus stroma cells in placenta of controls, women with preeclampsia and preterm delivery. Significantly differentially expressed peptides (p<0.01) and total number of peptides detected are described in italics, respectively.

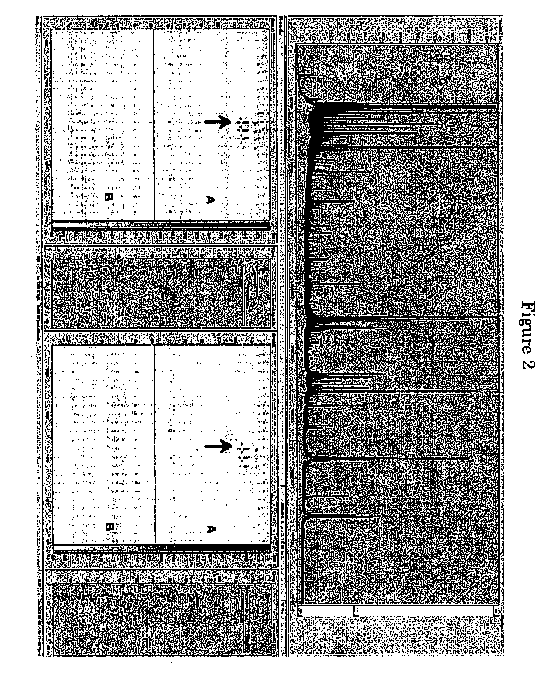

Fig. 2 provides a gel view presentation of the comparison of controls (n=7) versus cases (preeclampsia) (n=7) as obtained in ClinProTools software (Bruker Daltonics, Germany). Average mass spectra of trophoblast cases (blue) and control trophoblasts (red) were compared (top panel). The bottom panel shows gel views of specific areas of the mass spectrum that display for instance significant differential expressed peptides at 1250 (bottom left) and 1464 (bottom right). The arrows and graphs adjacent the gel views indicate the expression levels of the various samples for 1250 and 1464 respectively, which were primarily observed in controls (A) and not in preeclampsia (B) in the various samples.

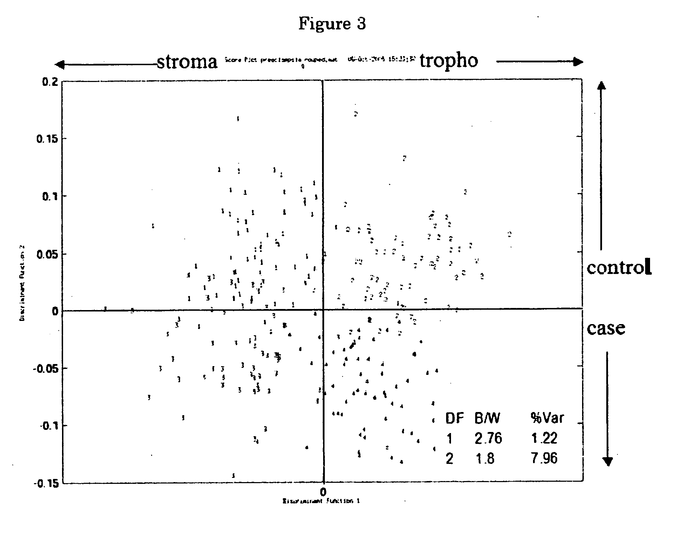

Fig. 3 depicts a representation of the discriminant analysis of controls (n=7) versus cases (preeclampsia) (n=7). DF1 describes the difference between trophoblasts and stroma (1.22 % variance) and DF2 describes the difference between control and case (7.96 % variance). A total of 255 spectra was included in this analysis. Note that the ratio of the between/within group variance (B/W) is > 1 for the 4 groups considered.

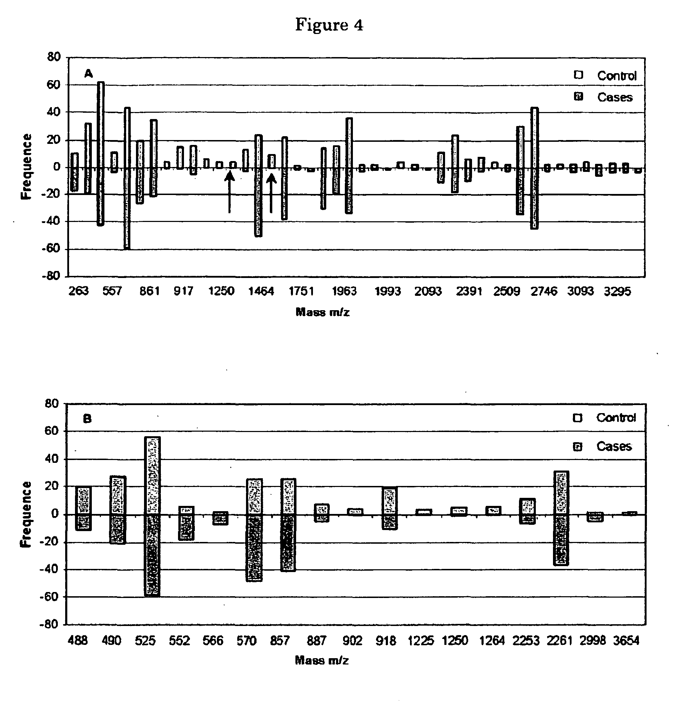

Fig. 4 depicts a representation of the frequency of differential expressed peptides between control (n=7) versus case trophoblasts (preeclampsia) (n=7) (A) and stroma cells (B), (p<0.01). Peptides that are significantly expressed will have a contribution from both differential intensities but also the frequency of occurrence in the two groups compared. Some peptide peaks are exclusively found in control or case trophoblast and stroma (arrows correspond to peptides 1250 and 1464 in Figure 2).

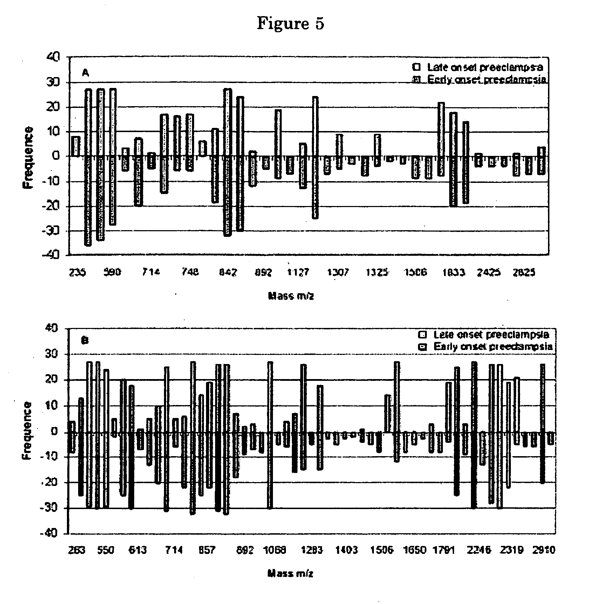

Fig 5. depicts a representation of the frequency of differential expressed peptides between early onset preeclampsia (n=4) versus late onset preeclampsia (n=3) trophoblasts (A) and stroma cells (B). A series of peptides that were exclusively found in early onset preeclamptics are observed.

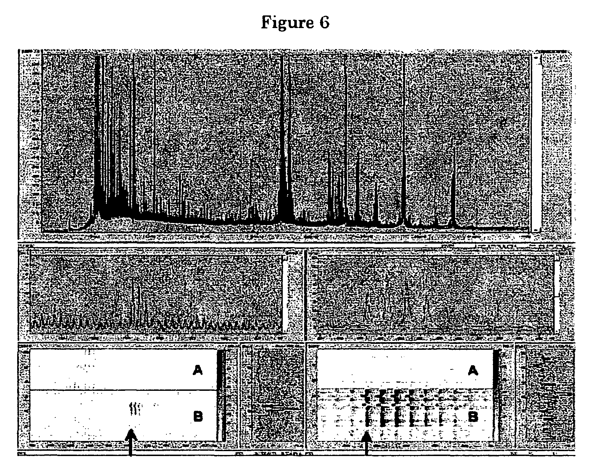

Fig. 6 provides a gel view presentation of the comparison of early onset preeclampsia versus preterm controls as obtained in ClinProTools software (Bruker Daltonics, Germany). Average mass spectra of early onset preeclampsia trophoblasts (blue) and preterm control trophoblasts (red) were compared (top panel). The bottom panel shows gel views of specific areas of the mass spectrum that display significant differential expressed peptides. The arrows illustrate peptides that were primarily observed in early onset preeclampsia (B) and not in preterm controls (A) (arrows correspond to peptides 1505 and 2691).

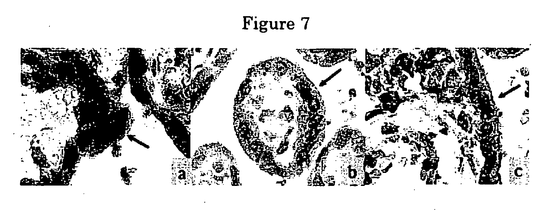

Fig. 7 illustrates immunohistochemistry of calcyclin in placenta of early onset preeclampsia (n=3) versus preterm and term controls (resp, n = 2 and n = 3). The arrows illustrate trophoblast cells. Preeclamptic trophoblast cells stain heavily with antibodies specific for calcyclin (S100A6) (panel A) in contrast to preterm and term controls (panel B and C, respectively). Some staining is observed in cells within the stroma of term controls (arrow heads; magnification 100X).

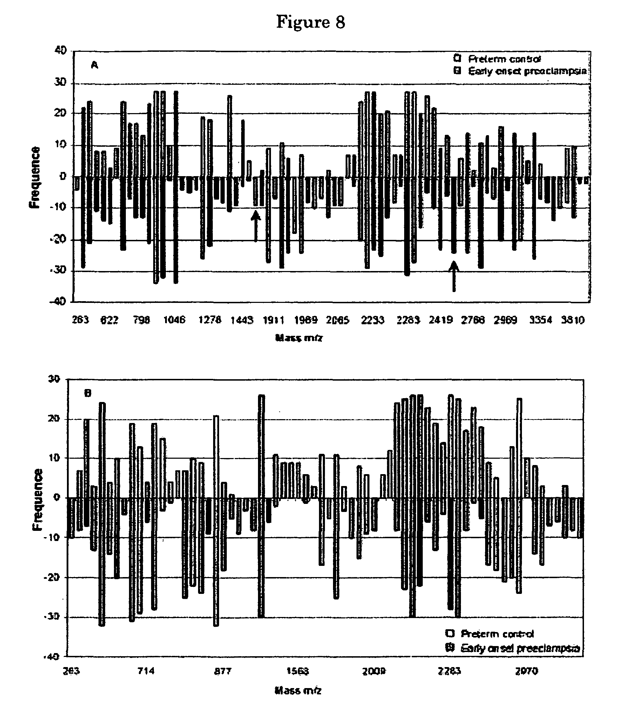

Fig. 8 depicts a representation of the frequency of differential expressed peptides between early onset preeclampsia (n=4) versus preterm controls (n=3) trophoblasts (A) and stroma cells (B). The arrows illustrate peptides, as an example, which were exclusively observed in early onset preeclampsia.

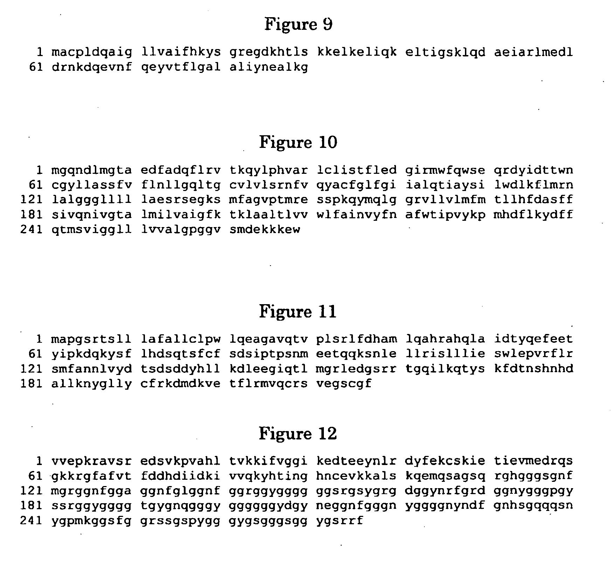

Fig. 9 shows the amino acid sequence of human calcyclin.
Fig. 10 shows the amino acid sequence of human surfeit locus protein.
Fig. 11 shows the amino acid sequence of human choriomammotropin A precursor.
Fig. 12 shows the amino acid sequence of human protein OTTHUMP00000018488.

### DETAILED DESCRIPTION OF THE PREFERRED EMBODIMENTS

The term "calcyclin," as used herein, refers to the member of the S100 family of proteins containing 2 EF-hand calcium-binding motifs localized in the cytoplasm and/or nucleus of a wide range of cells, and involved in the regulation of a number of cellular processes such as cell cycle progression and differentiation, also known as S100 calcium-binding protein A6 (S100A6), prolactin receptor-associated protein (PRA), Growth factor-inducible protein 2A9, and MLN 4 (UniProtKB/Swiss-Prot entry P06703). The term "calcyclin", as used herein, refers in particular to the human protein essentially having the amino acid sequence as shown in Fig. 9. The skilled person will understand that deviations and mutation may occur within the amino acid sequence or gene sequence of calcyclin, which deviations and mutations are encompassed in the term calcyclin as used herein. Also, the skilled person will understand from this specification that peptide fragments of the calcyclin protein can also be detected and serve as markers, such as peptides of the protein obtained by enzymatic (tryptic) digestion of samples of a subject wherein said marker is to be detected. In particular, suitable peptide fragments are fragments of calcyclin having a length of 7-100 amino acids, preferably 10-30 amino acids.

The term "surfeit locus protein" as used herein refers to the protein essentially having the amino acid sequence as shown in Fig. 10. When reference is made to the marker, peptide fragments of the surfeit locus protein can also be detected and serve as markers, such as peptides of the protein obtained by enzymatic (tryptic) digestion of samples of a subject wherein said marker is to be detected. In particular, suitable peptide fragments are fragments of surfeit locus protein having a length of 7-100 amino acids, preferably 10-3d amino acids.

The term "choriomammotropin A precursor" as used herein refers to the protein essentially having the amino acid sequence as shown in Fig. 11. When reference is made to the marker, peptide fragments of the choriomammotropin A precursor protein can also be detected and serve as markers, such as peptides of the protein obtained by enzymatic (tryptic) digestion of samples of a subject wherein said marker is to be detected. In particular, suitable peptide fragments are fragments of choriomammotropin A precursor having a length of 7-100 amino acids, preferably 10-30 amino acids.

The term "OTTHUMP00000018488" as used herein refers to the protein essentially having the amino acid sequence as shown in Fig. 12. When reference is made to the marker, peptide fragments of the protein OTTHUMP00000018488 can also be detected and serve as markers, such as peptides of the protein obtained by enzymatic (tryptic) digestion of samples of a subject wherein said marker is to be detected. In particular, suitable peptide fragments are fragments of OTTHUMP00000018488 having a length of 7-100 amino acids, preferably 10-30 amino acids.

The term "preeclampsia" refers to a medical condition where hypertension arises in pregnancy (pregnancy-induced hypertension) in association with significant amounts of protein in the urine. The term refers to a set of symptoms rather than any causative factor, and it is established that there can be many different causes for the syndrome. While blood pressure elevation is the most visible sign of the disease, it involves generalized damage to the maternal endothelium, kidneys and liver, with the release of vasopressive factors only secondary to the original damage. Preeclampsia may develop from 20 weeks gestation. It is defined as "early onset" when occurring before 34 weeks of gestation, which is associated with increased morbidity, i.e. involves the greatest risk for the foetus. It is defined as "late onset" when occurring after 34 weeks of gestation.

The term "chorionic villi" as used herein refers to are villi that sprout from the chorion, a structure consisting of two layers: an outer formed by the primitive ectoderm or trophoblast, and an inner formed by the somatic mesoderm; with this latter the amnion is in contact. The trophoblast is made up of an internal layer of cubical or prismatic cells, the cytotrophoblast or layer of Langhans, and an external layer of richly nucleated protoplasm devoid of cell boundaries, the syncytiotrophoblast. Chorionic villi as defined herein include primary, secundairy and tertiary chorionic villi, as well as floating villi, floating freely in the intervillous space, and anchoring (stem) villi, acting to stablise mechanical integrity of the placental-maternal interface.

The term "a sample of chorionic villi" refers to a sample obtained by chorionic villus sampling (CVS), including such techniques as transabdominal and transcervical sampling. Such a technique is suitable to be performed at early stage in pregnancy (i.e. before 34 weeks).

The term "determining the expression level of the protein" as used herein refers to measuring absolute or relative amounts of protein, and to absolute or relative amounts of transcription products transcribed from the genes encoding said protein. Said measuring involves measurements *"in vitro".*

### Markers for detecting early-onset preeclampsia in a patient

The present inventors identified several proteins that were specifically expressed in trophoblast cells from women with early onset preeclampsia relative to trophoblast cells in women with preterm delivery due to other or unknown cause (see Example 1) by using a method comprising the following steps:
(a) providing an optionally processed (e.g. trypsin digested) sample of trophoblast cells from women with early onset preeclampsia as a test sample, and an optionally processed sample of a corresponding cells of women with preterm delivery as a reference sample, wherein said samples comprise peptides and/or proteins;
(b) subjecting both test and reference sample to MALDI- FT-ICR mass spectrometry to generate mass spectra for individual peptides in each sample and to quantify the amount of individual peptides present in each sample;
(c) comparing the amount of an individual peptide present in the test sample with the amount of a peptide having a corresponding mass in the reference sample to generate a list of peptides differentially expressed between test and reference sample, and
(d) subjecting the test and/or reference sample of step (a) to tandem mass spectrometry (MS-MS), in order to identify the differentially expressed peptides and/or the proteins from which they derive thus providing a candidate marker protein or marker peptide.

In this method, microdissected chorionic villi biopsies were used. The peptides of the enzymatically digested proteins derived from the small numbers of cells obtained by microdissection, were measured by MALDI-FT mass spectrometry. The identification of differentially expresses peptides was achieved by combining nano-LC fractionation of samples with offline MALDI-TOF/TOF and MALDI FTMS measurements. The findings were validated by using specific antibodies. Details of these experiments are described in the Example below.

By using the above method, the inventors discovered a proteinaceous marker, calcyclin, the expression level of which was indicative for early onset preeclampsia.

In addition to calcyclin, also identified as markers indicative for early onset preeclampsia were surfeit locus protein, human protein OTTHUMP00000018:488 and choriomammotropin A precursor, and these markers may be used in addition to calcyclin in exactly the same manner.

The markers of the present invention are very suitably used in a method for monitoring the disease activity of early-onset preeclampsia or the response of the patient to treatment regimens aimed at ameliorating early-onset preeclampsia. Such a method comprises the step of measuring the expression level of for instance calcyclin in tissue. Reference values for markers may be determined as described below using for instance (MALDI) Triple-quad analysis and methods of diagnosis of preeclampsia may be performed as described in the Examples below.

Generally, the peptides of the marker calcyclin are detected with a mass accuracy of around 0.4 and 4 ppm when using the Ultimate NanoLC system and MALDI TOF/TOF method as described in the Example. The skilled person will understand that the exact amounts will depend on the marker detected, on the sample type, and on the reference values measured in samples obtained from normal, healthy subjects. The skilled artisan is well aware of methods to obtain reference values for diagnostic markers. Generally, typical reference samples will be obtained from subjects that are clinically well documented and that are free from the disease. In such samples, normal (reference) concentrations of the marker proteins can be determined, for instance by providing the average concentration over the reference samples. In determining the reference concentration of the marker a variety of considerations is taken into regard. Among such considerations are the type of disease to be diagnosed, the location of disease and the type of sample involved, the patient's age, weight, time in gestation, general physical condition and the like. For instance, a group of at least 2 to preferably more than 3 subjects, preferably ranked according to the above considerations, for instance from various age categories, are taken as reference group.

The marker of the present invention is absent in samples wherein no preeclampsia is present. In contrast, the marker is present in samples wherein preeclampsia is confirmed. For instance in the case of early onset preeclampsia, the calcyclin protein can easily be detected at elevated levels in the trophoblast cells of chorionic villi of women with preeclamsia at 13 weeks in gestation by immunological techniques, whereas in chorionic villi of healthy subjects, said marker is present at markedly lower concentrations or completely absent (See Fig. 7 and the Example).

In general, a level in the concentration of the marker that is increased at least 1.1-10 times, preferably 1.5-5 times, but suitably about 2 times, relative to concentration of the reference value is indicative of the presence of preeclampsia.

Depending on the normal (healthy) status, a marker indicative of preeclampsia as defined herein may be present in the diseased condition vs. absent in the normal condition, this is for instance the case with calcyclin. More often however, the level of expression of the marker will be altered, usually enhanced, so that elevated levels of the marker indicate the presence of the disease or even the severity of the disease condition, this is for instance the case for surfeit locus protein and ch. Therefore, in some instances, quantitative detection of the marker and comparison with reference values is necessary in order to draw conclusions. The steps which must be taken in order for a diagnosis to be made are generally:
i) an examination phase involving the collection of data,
ii) a comparison of these data with standard values,
iii) a finding of any significant deviation during the comparison, and
iv) the attribution of the deviation to a particular clinical picture, i.e. the deductive medical or veterinary decision phase.

In methods of the present invention, step iv is generally excluded. The methods of the present invention in particular relate to the technical steps of providing samples and providing clinical data on marker concentrations, which steps precede the deductive medical or veterinary decision phase.

Detection of the marker in a patient sample may be performed by any method available to the artisan. Generally, in order to detect the subtle concentration differences in the expression level of the marker, sophisticated methods are required. The skilled person is well acquainted with the various methods available, and these need not be described in great detail here.

In short, suitable methods include mass spectrometric methods such as those described and used herein, in particular in the Examples, and immunological detection methods.

Immunological detection methods (i.e. immunoassays) for determining the (quantitative) presence of a peptide or protein in a sample are well known to those of skill in the art. The markers identified by methods of the present invention can be employed as immunogens for constructing antibodies immunoreactive to a protein of the present invention for such exemplary utilities as immunoassays or protein purification techniques.

Polyclonal and monoclonal antibodies raised against calcyclin protein or peptide fragments thereof and that bind specifically thereto can be used for detection purpose in the present invention, for example, in immunoassays in which they can be utilized in liquid phase or bound to a solid phase carrier. In addition, the monoclonal antibodies in these immunoassays can be detectably labeled in various ways. A variety of immunoassay formats may be used to select antibodies specifically reactive with a particular peptide or protein marker. For example, solid-phase ELISA immunoassays are routinely used to select monoclonal antibodies specifically immunoreactive with a protein. See Harlow and Lane, Antibodies, A Laboratory Manual, Cold Spring Harbor Publications, New York (1988), for a description of immunoassay formats and conditions that can be used to determine selective binding. Examples of types of immunoassays that can utilize monoclonal antibodies of the invention are competitive and non-competitive immunoassays in either a direct or indirect format. Examples of such immunoassays are the radioimmunoassay (RIA) and the sandwich (immunometric) assay.

Detection of the peptide or protein marker using an antibody can be done utilizing immunoassays that are run in either the forward, reverse, or simultaneous modes, including immunological assays on physiological samples. Those of skill in the art will know, or can readily discern, other immunoassay formats without undue experimentation.

Immunological detection may for instance be performed by ELISA using commercial tests for detection of calcyclin in for instance CSF samples (DakoCytomation, Denmark).

Antibodies can be bound to many different carriers and used to detect the presence of the disease markers. Examples of well-known carriers include glass, polystyrene, polypropylene, polyethylene, dextran, nylon, amylases, natural and modified celluloses, polyacrylamides, agaroses and magnetite. The nature of the carrier can be either soluble or insoluble for purposes of the invention. Those skilled in the art will know of other suitable carriers for binding monoclonal antibodies, or will be able to ascertain such using routine experimentation.

The binding of the antibody to the marker of the present invention can be detected in numerous ways that are well known in the art. Binding of the antibody and disease marker forms an immune complex that can be detected directly or indirectly. The immune complexes are detected directly, for example, when the antibodies employed are conjugated to a label. The immune complex is detected indirectly by examining for the effect of immune complex formation in an assay medium on a signal producing system or by employing a labeled receptor that specifically binds to an antibody. Suitable detection techniques that may be applied in concert with the above techniques include autoradiographic detection techniques, detection techniques based on fluorescence, luminescence or phosphorescence or chromogenic detection techniques. These detection techniques are known in the art of detection of biomolecules.

Use may for instance be made of signal producing systems, involving one or more components, at least one component being a detectable label, which generate a detectable signal that relates to the amount of bound and/or unbound label, i.e. the amount of label bound or not bound to the compound being detected. The label is any molecule that produces or can be induced to produce a signal, and preferably is a fluorescer, radio-label, enzyme, chemiluminescer or photosensitizer. Thus, the signal is detected and/or measured by detecting fluorescence or luminescence, radioactivity, enzyme activity or light absorbance.

Suitable labels include, by way of illustration and not limitation, enzymes such as alkaline phosphatase, glucose-6-phosphate dehydrogenase ("G6PDH") and horseradish peroxidase; ribozyme; a substrate for a replicase such as QB replicase; promoters; dyes; fluorescers, such as fluorescein, rhodamine compounds, phycoerythrin, phycocyanin, allophycocyanin, o-phthaldehyde, and fluorescamine; chemiluminescers such as isoluminol; sensitizes; coenzymes; enzyme substrates; radiolabels such as ¹²⁵I, ¹⁴O, ³H, ⁵⁷Co and ⁷⁵Se; particles such as latex or carbon particles; metal sol; crystallite; liposomes; cells, etc., which may be further labeled with a dye, catalyst or other detectable group. Suitable enzymes and coenzymes are disclosed in U.S. Patent No. 4,275,149; U.S. Patent No. 4,318,980; suitable fluorescers and chemiluminescers are disclosed i.a. in U.S. Patent No. 4,275,149.

There are numerous methods by which the label can produce a signal detectable by external means, for example, desirably by visual examination or by electromagnetic radiation, heat, and chemical reagents. The label or other signal producing system component can also be bound to a specific binding partner, another molecule or to a support.

The label can directly produce a signal, and therefore, additional components are not required to produce a signal. Numerous organic molecules, for example fluorescers, are able to absorb ultraviolet and visible light, where the light absorption transfers energy to these molecules and elevates them to an excited energy state. This absorbed energy is then dissipated by emission of light at a second wavelength. Other labels that directly produce a signal include radioactive isotopes and dyes.

Alternately, the label may need other components to produce a signal, and the signal producing system would then include all the components required to produce a measurable signal, which may include substrates, coenzymes, enhancers, additional enzymes, substances that react with enzymic products, catalysts, activators, cofactors, inhibitors, scavengers, metal ions, and a specific binding substance required for binding of signal generating substances. A detailed discussion of suitable signal producing systems can be found in U.S. Patent No. 5,185,243.

The label can be bound covalently to numerous specific binding partners: an antibody; a receptor for an antibody; a receptor that is capable of binding to a small molecule conjugated to an antibody; or a ligand analog. Bonding of the label to the specific binding partner may be accomplished by chemical reactions which result in replacing a hydrogen atom of the label with a bond to the specific binding partner member or may include a linking group between the label and the specific binding partner. Other signal producing system components may also be bound covalently to specific binding partners. For example, two signal producing system components such as a fluorescer and quencher can each be bound to a different antibody that forms a specific complex with the analyte.

Formation of the complex brings the fluorescer and quencher in close proximity, thus permitting the quencher to interact with the fluorescer to produce a signal. Methods of conjugation are well known in the art. See for example, U.S. Patent No. 3,817,837.

An antibody may also be bound to a first signal producing system component and a detectable label as the second signal producing system components. For example, when the detectable label is bound to a ligand analog, the extent of binding of the antibody to the analog can be measured by detecting the signal produced by the interaction of the signal producing system components.

Methods and means provided herein are particularly useful in a diagnostic kit for diagnosing a disease by immunological techniques. Such kits or assays may for example comprise one or more reference markers, one or more reference samples and/or one or more antibodies for any of the markers for the various disease conditions as described herein, and can be used specifically to carry out a method or use.

Methods for measuring the expression level of peptides or proteins by MALDI techniques as referred to herein are well known in the art and specific reference is made to the Experimental part described herein below.

The invention will now be illustrated by the following non-limiting Examples.

### EXAMPLES

### Example 1. Specific peptides identified by mass spectrometry in placental tissue from pregnancies complicated by early onset preeclampsia attained by laser capture dissection

### 1.1. Experimental Procedures and Methods

### 1.1.1. Placental samples

A total of 17 human placentas were obtained at the obstetrical wards of the departments of Obstetrics and Gynaecology of the Radboud University Nijmegen Medical Center, Nijmegen, and Erasmus MC Rotterdam, The Netherlands, after having given informed consent. Of these 17 placentas 7 were obtained from women after uncomplicated normotensive pregnancies, 7 from women with preeclampsia and 3 placentas from normotensive women after preterm delivery of unknown cause (excluding infection or systemic diseases). From the 7 women with preeclampsia, 4 experienced early onset preeclampsia (before 34 weeks gestation) and 3 late onset preeclampsia (Table 1).

**Table 1. Clinical characteristics of pregnancies from studied placenta samples.**

| | Diagnosis | Maternal age (yr) | Parity | Blood pressure (mm Hg)* | Proteinuria (g/10 mmol) | Gestational age at delivery (days) | Birth weight (g) | Fetal sex |
|---|---|---|---|---|---|---|---|---|
| 1 | E-PE | 31 | 0 | 110 | 47 | 239 | 1920 | M |
| 2 | E-PE | 28 | 0 | 115 | 6.63 | 219 | 909 | F |
| 3 | E-PE | 32 | 1 | 120 | 21,11 | 236 | 1856 | M |
| 4 | E-PE | 33 | 0 | 115 | 5.51 | 213 | 1053 | F |
| 5 | PE | 26 | 0 | 120 | 0.79 | 268 | 3045 | M |
| 6 | PE | 26 | 0 | 115 | 7,11 | 276 | 2580 | M |
| 7 | PE | 20 | 0 | 130 | 6,2 | 254 | 2343 | M |
| 8 | NL | 22 | 1 | 75 | 0 | 269 | 3025 | F |
| 9 | NL | 33 | 0 | 75 | 0 | 268 | 3010 | F |
| 10 | NL | 32 | 1 | 67 | 0 | 271 | 3220 | M |
| 11 | NL | 31 | 1 | 80 | 0 | 274 | 3370 | M |
| 12 | NL | 30 | 0 | 70 | 0 | 277 | 2335 | M |
| 13 | NL | 30 | 0 | 80 | 0 | 264 | 3150 | F |
| 14 | NL | 27 | 2 | 75 | 0 | 274 | 4110 | M |
| 15 | Preterm | 24 | 0 | 100 | 0 | 204 | 1110 | M |
| 16 | Preterm | 21 | 0 | 65 | 0 | 223 | 1750 | M |
| 17 | Preterm | 38 | 2 | 70 | 0 | 218 | 1695 | F |

| | | | | | | | | |
|---|---|---|---|---|---|---|---|---|
| Diagnosis: E-PE = early onset preeclampsia, PE = preeclampsia, NL = normal, Preterm = preterm delivery, * Blood pressure = diastolic blood pressure at admission, proteinuria = protein/creatine ratio, normal 0.3 g/10 mmol, F = female, M = male | | | | | | | | |

Women with early onset preeclampsia were matched for gestational age and parity with women with preterm delivery of unknown cause. One woman in the last mentioned group had hypertension at admission (#15, pregnancy induced hypertension at admission, no preeclampsia). Preeclampsia was defined according to the International Society for the Study of Hypertension in Pregnancy (ISSHP) definition (Am. J. Obstet. Gynecol. 183, S1-22 (2000)). The Medical Ethics Review Boards of the Radboud University Nijmegen Medical Center and the Erasmus MC, Rotterdam approved the protocol.

### 1.1.2. Laser microdissection

Placenta trophoblastic tissues were dissected as described recently (De Groot, C.J., et al. (2005) Proteomics 5, 597-607). In brief, placenta sections were directly frozen in liquid nitrogen and liquid iso-pentane (Brunschwig Chemie, Amsterdam, the Netherlands). The frozen tissue was embedded into Cryoblock (Klinipath BV, Duiven, Netherlands) tissue medium and subsequently frozen tissue sections (5 µm) were made by cryostat (type HM 500 Adamas, Rhenen, The Netherlands). The sections were mounted on a PEN (polyethylene naphthalate) membrane (1.35 µm) as recommended by the manufacturer (P.A.L.M. Microlaser Technologies AG, Bernried, Germany). After cutting, the sections were placed in 70 % alcohol (99,8 % purity) for 1 minute followed by 10 seconds in Milli-Q water, 1 minute in hematoxylin (2x repeated), 1 minute in 0.5% acetic acid, 1 minute in ammonia water, and eosine for 10 seconds, respectively. Subsequently, the slides were dehydrated in 96 % alcohol and 100 % alcohol each for 1 minute, respectively. Dehydration with 96 % alcohol and 100 % alcohol were repeated twice.

Trophoblast cells were captured with a P.A.L.M Microlaser capture microdissection (type P-MB, Bernried Germany). The microdissected cells were collected in 0.1 % Rapigest SF reagent (sodium 3-[(2-methyl-2-undecyl-1,3-dioxolan-4-yl)methoxy]-1-propanesulfonate) (Waters Corporation, Milford, MA, USA) in 50 mM ammonium bicarbonate buffer for approximately 1:5 hour before digestion with trypsin.

Laser microdissected tissues were centrifuged at 10,000 rpm for 10 seconds and subsequently sonified by a Ultrasonic Disruptor Sonifier II Model W-250/W-450 (Bransons Ultrasonics) equipped with a cuphorn for 1 minute at 70% amplitude. Samples were boiled for 5 minutes. After cooling to room temperature, trypsin (Promega Corporation Cat No.: V5280, U.S.A.) solutions were added routinely at a concentration of 0.1 ug/ uL. Digestion was performed for 1.5 hour at 37°C. Subsequently, the digestion reaction was stopped by incubating at 50 mM HCl.

### 1.1.3. Mass spectrometry measurements

Mass spectrometry was automatically performed on an Ultraflex I MALDI-TOF mass spectrometer (Bruker Daltonics, Billerica, USA), with a laser power of 50 - 60 % and 1,000 laser shots per single spectrum. Calibration occurred with the peptide calibration standard (Bruker Daltonics Part No.: 206195, Bremen, Germany). The control spectra, obtained from sole PEN membrane treated in the same way as microdissected tissue, were used as background spectra. Independently, microdissection was performed three times and from each sample a triplicate mass spectrometry measurement was performed. Accordingly, 9 spectra were obtained from each of the 17 placentas. The reproducibility of the intensities of the measurements of the complex peptide mixtures ranged between 15% and 30%.

### 1.1.4. Data and Mass spectrometry analysis

Analysis of mass spectra was performed by using ClinProTools biosoftware (version 1, Bruker Daltonics, Leipzig, Germany) using comparisons illustrated in Figure 1. The proteome data of the trophoblast and stroma data were compared between placentas from women with uncomplicated pregnancies (controls, n=7), women with pregnancies complicated by late (n=3) and early onset preeclampsia (n=4), and placentas from women with unexplained preterm birth (n=3). Significant differences of monoisotopic peaks were defined as p < 0.01. The differentially expressed peaks excluding the background peaks of the PEN membrane were mass annotated.

Discriminant analysis and Principal Component Analysis (PCA) of the MALDI-TOF data was performed using ChemoMetricks, an in-house developed statistical analysis toolbox. This toolbox was developed on the Matlab 5.2 (Mathworks, Nantucket MA) platform. All spectra were truncated to 190700 points. Individual spectra were subsequently rebinned in segments of 100 points, yielding 122 spectra of 1907 variables for the preterm data and 255 spectra with 1907 variables in the 7 control versus 7 cases comparison. After reading in the full data the data was normalized and autoscaled prior to PCA. After computing the Principal Components (PC) and the Discriminant functions (DF) all data was plotted using standard Matlab plotting routines.

Placental tissue sections of the samples (3, 10, 17, Table 1) representative for the three groups were separated on an Ultimate NanoLC system (Dionex, Sunnyvale, CA, USA) prior to MALDI-TOF analysis. Assuming that a cell size is 10 x 10 x 10 µm, the tissue represented approximately 50.000 cells. Tissue samples were prepared in the same way as microdissected tissue. Subsequently, fractionation was performed on 5 µL tissue sample solutions, for 130 min with a 5 to 95% step wise gradient of solution A (100% H2O, 0.05% TFA) and solution B (20% H2O, 80% ACN, 0.04% TFA). A 15 cm C18 column with 75 µm ID and a guard column (Dionex, Sunnyvale, CA, USA) were used. Separate fractions were collected using a robotic spotter (Probot Micro Fraction collector, Dionex, Sunnyvale, CA, USA) on a prespotted, with matrix of α-cyano-4-hydroxycinnimic acid, anchorchip plate (Bruker Daltonics Germany). One µL of each fraction was spotted. Mass spectrometry analyses were performed in reflectron mode using standard settings for peptide measurements (Bruker default file "Proteomics_HPC.par") in addition to the MS spectrum a MS/MS spectrum was obtained for each peak using the standard settings for a lift MS/MS measurement. LC Warp software (Bruker Daltonics) was used to combine MS/MS spectra of peptides of the same protein. The resulting spectra were analyzed and annotated in Flexanalyses 2.2 (Bruker Daltonics). In Biotools 2.2 (Bruker Daltonis) the peak lists from the MS spectra and the MS/MS were combined to create a MGF file. The MGF file was used as input for the Mascot search engine (Matrix Science, London, UK) to search the MSDB human database using a 100 ppm tolerance of the MS spectra and a 0.5 Da tolerance for the fragments in the MS/MS spectra. Identification was significant, if a Mowse score with a probability of p<0.05 was observed in the Mascot search engine.

### 1.1.5. MALDI FT ICR mass spectrometry

Prespotted anchorchip plates (Bruker Daltonics, Germany) used for the MS/MS sequencing in the MALDI TOF/TOF were spotted with DHB (10 mg/ mL 0.1%TFA water). Subsequently, for confirmation remains of the fractions were analyzed by MALDI FT ICR MS (APEX Q, Bruker Daltonics) for accurate mass annotation. Identification was confirmed if the calculated mass of the identified differential expressed peptide peaks observed in the MALDI-TOF/TOF analysis did not deviate more than 4 ppm in the MALDI FT-ICR MS analysis from the calculated mass of the peptide identified.

### 1.1.6. Validation of calcyclin and choriomammotropin by immunohistochemistry

Commercial available antibodies specific for calcyclin (P06703, Sigma Aldrich, Saint Louis, USA) and choriomammotropin (LCHUC, DakoCytomation, Glostrup, Denmark) were used for validation by immunohistochemistry: according to the recommendation of the manufacturers.

### 1.2. Results

### 1.2.1. Clinical data

Clinical and demographic characteristics are given in table 1. As defined women with preeclampsia (n=7; # 1 - 7) had significantly higher blood pressure at admission and proteinuria. As expected these women also had on average a shorter gestational age (p< 0.05) and their newborns had a lower birth weight (p<0.05) compared to women with uncomplicated pregnancies (n=7; #8-14). No significant differences were found between women with preeclampsia and controls regarding maternal age and parity. For detailed analysis preeclampsia was stratified into early onset preeclampsia (n=4; # 1-4) and late onset preeclampsia (n=3; # 5-7). Between women with severe early onset preeclampsia (n=4) and women with preterm delivery of unknown cause (n=3) no differences were found for gestational age at delivery and birth weight. All but one woman were delivered by caesarean section (# 16).

### Analysis of peptide spectra of placentas from controls and preeclamptics

In Figure 1 numbers of significantly differentially expressed peptides and total numbers of peptides for various comparisons are given for trophoblast and villous stroma cells of women with uncomplicated pregnancies, early- and late onset preeclamptic women and women with preterm delivery. In Figure 2 differences between peptide profiles of trophoblasts of placentas from women with uncomplicated pregnancy (n=7) and preeclampsia (n=7) are illustrated in different views. From each mass over charge value (m/z) a statistical evaluation is performed using ClinProTools software (Bruker Daltonics, Leipzig, Germany). In tables 2 and 3, the exact data of significantly expressed peptides (p<0.01) are described for both the comparison of trophoblast and stroma cells, respectively.

**Table 2. Differences in peptides of trophoblast cells from placentas of preeclamptics (n=7) and controls (n=7).**

| mass | Ave control | Ave case | SD control | SD case | p-value | t-test | Spectra controls 1) | Controln 2) | Subsample a control | Spectra cases1) | Patient cases2) | Subsampl es cases |
|---|---|---|---|---|---|---|---|---|---|---|---|---|
| 263.082 | 16.3631 | 8.17539 | 21.1839 | 6.17205 | 0.00258327 | 3.10687 | 10 | 4 | 4/2/4 | 17 | 6 | 7/3/7 |
| 487.841 | 38.5705 | 20.8703 | 34.3616 | 11.5647 | 9.89E-05 | 4.07983 | 32 | 7 | 11/10/11 | 19 | 5 | 8/5/6 |
| 531.077 | 46.453 | 38.0458 | 16.8788 | 12.0523 | 0.00115908 | 3.32484 | 62 | 7 | 18/20/22 | 42 | 7 | 13/15/14 |
| 557.41 | 28.7307 | 21.4808 | 12.9487 | 8.67216 | 0.000207529 | 3.82398 | 11 | 4 | 4/6/1 | 4 | 2 | 2/1/1 |
| 570.232 | 71.805 | 86.1616 | 30.0831 | 31.0658 | 0.00820695 | -2.6861 | 44 | 6 | 14/17/13 | 59 | 7 | 20/24/15 |
| 646.784 | 22.3257 | 28.7206 | 12.9758 | 12.4333 | 0.00456151 | -2.8872 | 44 | 4 | 5/7/8 | 26 | 6 | 11/8/7 |
| 861.148 | 27.7616 | 20.388 | 13.1927 | 11.7102 | 0.000891299 | 3.40144 | 20 | 5 | 10/13/11 | 21 | 6 | 7/9/5 |
| 887.841 | 29.0736 | 14.299 | 23.8716 | 4.69182 | 2.39E-06 | 5.10167 | 4 | 2 | 1/1/2 | 1 | 1 | 1/0/0 |
| 901.488 | 20.4772 | 11.5813 | 17.8979 | 2.28037 | 9.96E-05 | 4.14907 | 15 | 4 | 4/4/7 | 1 | 1 | 0/1/0 |
| 917.284 | 25.6835 | 15.3069 | 19.8261 | 8.24842 | 0.00114068 | 4.02339 | 16 | 3 | 7/6/3 | 5 | 3 | 0/3/2 |
| 1119.29 | 11.995 | 8.94666 | 8.2868 | 2.96339 | 0.00480048 | 2.89186 | 6 | 2 | 1/2/3 | 0 | 0 | 0/0/0 |
| 1223.79 | 9.27621 | 7.10801 | 4.02103 | 2.26607 | 0.000174418 | 3.88226 | 4 | 1 | 0/3/0 | 0 | 0 | 0/0/0 |
| 1249.87 | 13.4275 | 7.46008 | 12.8263 | 3.3023 | 0.000302125 | 3.7725 | 4 | 1 | 1/2/1 | 0 | 0 | 0/0/0 |
| 1263.7 | 13.6549 | 9.11307 | 10.1092 | 3.28615 | 0.000586235 | 3.57236 | 13 | 4 | 2/5/6 | 3 | 1 | 0/0/3 |
| 1274.76 | 20.0197 | 40.2306 | 15.5879 | 28.6243 | 3.87E-06 | 4.923 | 24 | 4 | 6/9/7 | 50 | 6 | 18/17/15 |
| 1463.9 | 9.67729 | 6.71123 | 7.91324 | 2.19671 | 0.00331065 | 3.02554 | 9 | 1 | 3/3/9 | 0 | 0 | 0/0/0 |
| 1529.8 | 13.9933 | 22.911 | 10.0955 | 15.1133 | 0.000161617 | -3.9182 | 22 | 5 | 6/7/7 | 38 | 6 | 13/14/11 |
| 1744.91 | 13.3673 | 17.7288 | 8.52165 | 10.1513 | 0.009197 | -2.6484 | 1 | 1 | 0/0/1 | 2 | 2 | 1/0/0 |
| 1750.98 | 6.7016 | 8.35852 | 2.10111 | 4.08152 | 0.00528678 | -2.8614 | 0 | 0 | 0/0/0 | 3 | 1 | 2/1/0 |
| 1833.96 | 14.334 | 19.944 | 10.3843 | 11.3303 | 0.00383763 | -2.9472 | 14 | 3 | 6/8/3 | 30 | 6 | 11/11/18 |
| 1922.99 | 11.6271 | 14.2023 | 5.33754 | 5.40957 | 0.00696017 | -2.7436 | 16 | 4 | 7/6/3 | 19 | 5 | 7/7/5 |
| 1962.99 | 15.3871 | 18.0859 | 6.05165 | 5.36474 | 0.0075167 | -2.7156 | 36 | 6 | 15/10/11 | 33 | 6 | 11/10/12 |
| 1969.04 | 8.7128 | 10.5099 | 3.37162 | 2.98614 | 0.00148358 | -3.2471 | 2 | 1 | 0/0/2 | 3 | 1 | 1/2/0 |
| 1989.02 | 8.0858 | 9.24187 | 2.47274 | 2.15234 | 0.00476722 | -2.8718 | 2 | 1 | 2/0/0 | 2 | 1 | 1/0/1 |
| 1993.04 | 6.4040 | 7.48408 | 2.04107 | 2.15957 | 0.00393622 | -2.9381 | 0 | 0 | 1/0/3 | 2 | 1 | 1/0/1 |
| 2003.04 | 7.47908 | 8.89717 | 2.6828 | 3.13957 | 0.00655682 | .2.7674 | 4 | 1 | 1/0/3 | 0 | 0 | 0/0/0 |
| 2065.08 | 6.4621 | 7.59931 | 1.62099 | 2.04548 | 0.000666571 | -3.4994 | 2 | 1 | 1/1/0 | 2 | 1 | 1/1/0 |
| 2093.12 | 5.7865 | 7.99298 | 1.28468 | 5.5112 | 0.00323833 | -3.0564 | 0 | 0 | 1/2/7 | 2 | 1 | 1/0/1 |
| 2149.11 | 6.1218 | 7.14392 | 1.31519 | 2.18626 | 0.00193221 | -3.1891 | 10 | 3 | 1/2/7 | 11 | 4 | 6/1/4 |
| 2312.2 | 7.58108 | 10.0398 | 2.92523 | 6.18476 | 0.00561813 | -2.8437 | 24 | 5 | 7/6/11 | 18 | 4 | 8/6/4 |
| 2391.28 | 7.53957 | 10.1508 | 3.0919 | 6.01408 | 0.00293531 | -3.0613 | 6 | 2 | 3/3/0 | 10 | 2 | 5/2/3 |
| 2424.31 | 8.09969 | 9.53876 | 3.61635 | 2.6154 | 0.0092417 | -2.6436 | 8 | 1 | 3/3/2 | 3 | 1 | 1/2/0 |
| 2435.5 | 7.48574 | 10.1287 | 3.02709 | 4.27602 | 0.000102918 | -4.0361 | 4 | 1 | 2/2/0 | 0 | 0 | 0/0/0 |
| 2509.27 | 6.21672 | 7.64788 | 2.38112 | 2.53189 | 0.00115145 | -3.3279 | 2 | 2 | 0/0/2 | 3 | 3 | 2/1/0 |
| 2676.35 | 8.50049 | 10.7379 | 3.54022 | 2.8841 | 0.000106862 | -3.9974 | 30 | 5 | 9/11/10 | 34 | 6 | 13/12/9 |
| 2690.34 | 8.8189 | 10.7772 | 4.00463 | 3.41302 | 0.00108296 | -3.343 | 44 | 5 | 15/15/14 | 45 | 6 | 17/16/12 |
| 2746.39 | 7.02622 | 8.58981 | 3.07062 | 2.45706 | 0.00148131 | -3.2479 | 2 | 2 | 0/0/2 | 3 | 2 | 0/0/3 |
| 2869.51 | 6.93271 | 8.27684 | 2.82235 | 2.96351 | 0.00894567 | -2.6557 | 2 | 1 | 2/0/0 | 1 | 1 | 1/0/0 |
| 2936.5 | 8.55322 | 10.1708 | 2.88537 | 2.47244 | 0.000709859 | -3.4687 | 2 | 2 | 2/0/0 | 4 | 2 | 3/0/1 |
| 3092.57 | 6.26394 | 7.88215 | 2.83176 | 2.45235 | 0.000598814 | -3.6184 | 4 | 2 | 2/0/2 | 3 | 1 | 1/1/1 |
| 3093.61 | 7.75564 | 9.646 | 5.48941 | 4.19775 | 0.00620724 | -2.7871 | 2 | 1 | 1/0/1 | 6 | 5 | 3/1/2 |
| 3293.8 | 7.77449 | 9.39953 | 3.25616 | 2.6201 | 0.00210387 | -3.1383 | 3 | 2 | 0/1/1 | 4 | 2 | 1/1/2 |
| 3294.8 | 8.70374 | 10.6677 | 3.78652 | 3.42341 | 0.00216987 | -3.1286 | 3 | 3 | 0/1/2 | 4 | 3 | 1/1/2 |
| 3493.87 | 5.75585 | 6.93194 | 2.49604 | 205888 | 0.00359315 | -2.9659 | 0 | 0 | 0/0/0 | 4 | 3 | 2/1/1 |

| | | | | | | | | | | | | |
|---|---|---|---|---|---|---|---|---|---|---|---|---|
| Ave= average; 1) = number of peptides observed in all spectra (the maximum is 63); 2) number of patients or controls that contributed; subsamples in x/x/x = measured in the first, the second and the third measurement. | | | | | | | | | | | | |

**Table 3. Differences in peptides of stroma cells from placentas of preeclamptics (n=7) and controls (n=7).**

| Ave mass | Ave control | Ave case | SD control | SD case | p-value | t-test | Spectra control a¹⁾ | Con-trols ²⁾ | Subsamples control | Spectra cases ¹⁾ | Patient cases ²⁾ | Subsam ples cases |
|---|---|---|---|---|---|---|---|---|---|---|---|---|
| 487.814 | 42.1178 | 23.1608 | 30.7113 | 11.0834 | 1.65E-05 | -4.59059 | 20 | 6 | 8/6/6 | 11 | 5 | 4/4/3 |
| 490.309 | 25.0365 | 12.6763 | 20.8517 | 4.06296 | 1.85E-05 | -4.61239 | 27 | 7 | 10/8/9 | 21 | 5 | 7/6/7 |
| 524.66 | 7.29572 | 8.82291 | 1.82001 | 2.07677 | 3.48E-05 | 4.30785 | 56 | 7 | 17/20/19 | 58 | 7 | 20/17/19 |
| 552.398 | 10.8553 | 7.8641 | 4.53703 | 3.11542 | 4.18E-05 | -4.2687 | 6 | 4 | 4/2/0 | 18 | 5 | 7/7/4 |
| 566.2 | 10.5989 | 13.6552 | 3.63932 | 4.3106 | 5.05E-05 | 4.21418 | 2 | 2 | 1/0/1 | 7 | 4 | 3/3/1 |
| 570.173 | 29.8944 | 16.5442 | 24.467 | 7.38558 | 9.29E-05 | -4.1344 | 26 | 6 | 8/8/9 | 48 | 7 | 18/14/15 |
| 856.671 | 10.2805 | 7.48291 | 5.34308 | 2.44612 | 0.000308589 | -3.7667 | 26 | 5 | 11/6/9 | 41 | 7 | 15/14/12 |
| 886.954 | 63.3734 | 84.9042 | 32.2283 | 33.5808 | 0.00451973 | 3.60859 | 7 | 3 | 1/3/3 | 5 | 2 | 2/2/1 |
| 902.397 | 36.7834 | 48.4946 | 18.1009 | 19.6097 | 0.00085833 | 3.92113 | 4 | 3 | 3/0/1 | 0 | 0 | 0/0/0 |
| 917.846 | 11.6403 | 7.68683 | 8.68136 | 3.19104 | 0.00112956 | -3.37908 | 19 | 6 | 6/6/7 | 10 | 4 | 5/3/2 |
| 1224.87 | 9.72086 | 7.31542 | 6.51566 | 2.53906 | 0.00801883 | -2.71815 | 3 | 2 | 2/0/1 | 0 | 0 | 0/0/0 |
| 1249.83 | 34.3887 | 41.2978 | 11.8057 | 11.7529 | 0.00155927 | 3.23779 | 5 | 1 | 3/0/2 | 1 | 1 | 1/0/0 |
| 1263.82 | 28.3048 | 17.1258 | 25.4219 | 9.09092 | 0.00157863 | -3.27395 | 6 | 3 | 1/0/5 | 0 | 0 | 0/0/0 |
| 2253.33 | 10.5989 | 13.6552 | 9.6392 | 4.31603 | 5.05E-05 | 4.21418 | 11 | 3 | 3/3/4 | 6 | 4 | 4/1/1 |
| 2261.21 | 10.8924 | 7.84545 | 7.56622 | 3.11925 | 0.004232 | -2.94093 | 31 | 6 | 12/8/11 | 37 | 6 | 14/11/12 |
| 2997.68 | 8.36354 | 6.97422 | 3.37358 | 1.88571 | 0.00563323 | -2.83051 | 2 | 1 | 0/0/2 | 5 | 2 | 1/4/0 |
| 3654.25 | 6.88494 | 8.51103 | 3.32037 | 3.23387 | 0.0070877 | 2.7398 | 2 | 2 | 1/0/3 | 0 | 0 | 0/0/0 |

| | | | | | | | | | | | | |
|---|---|---|---|---|---|---|---|---|---|---|---|---|
| Ave= average; ¹⁾ = number of peptides observed in all spectra; ²⁾ number of patients or controls that contributed; subsamples in x/x/x = measured in the first, the second and the third measurement. | | | | | | | | | | | | |

Discriminant analysis revealed that the major spectral differences have to be attributed to patient variation. Approximately 17 % of the data variance is attributed to patient variation. The classification by cell-type and control/case variance contributes only 11% to the full data set as can be seen in figure 3 where DF1 describes the difference between trophoblasts and stroma (1.22 % variance) and DF2 describes the difference between control and case (7.96 % variance). Figure 3 demonstrates that under these constraints the different groups of preeclampsia and controls can be clearly separated.

The PCA and DA of MALDI-TOF peptide patterns of trophoblasts as well as stroma cells of placentas both from women with preeclampsia as well as controls are separated significantly. Ten samples were misclassified in the trophoblast vs. stroma classification. The control vs. case separation contained 15 misclassified spectra out of the 255 total spectra, i.e. approximately 6%. In Figure 4 the frequency of the significantly differentially expressed peptides (as function of presence and intensity) in trophoblasts and villous stroma cells in placentas from women with uncomplicated pregnancies (n=7) and preeclampsia (n=7) are displayed.

### 1.2.2. Analysis of peptide spectra of placentas from early onset preeclamptics and unexplained preterm delivery

Figure 5 illustrates the frequency of differentially expressed peptides in women with early compared to late onset preeclampsia. In order to determine whether the differences between differentially expressed peptides of women with early vs. late onset preeclampsia could be explained by the maturity of the placenta, differences in peptide profiles of trophoblast cells between early onset preeclampsia and preterm delivery with unknown cause are illustrated in Figure 6.

We observed that the m/z values (e.g. 1269,76; 1505,81; 1910,99; 1969,01; 2044,01; 2691,3) were specific for early onset preeclampsia trophoblast samples (Figure 8). In Tables 4 and 5 the data of significantly expressed peptides and their presence in trophoblast and villous stroma cells is described in detail.

**Table 4. Significant differences in peptides of trophoblasts from placentas from early onset preeclampsia (n=4) and preterm delivery (n=3).**

| Ave mass | Ave control | Ave case | SD control | SD case | p-value | t-test | Spectra controls ¹⁾ | Controls ²⁾ | Subsample s control | Spectra cases ¹⁾ | Patient cases ²⁾ | Subsampt es cases |
|---|---|---|---|---|---|---|---|---|---|---|---|---|
| 262.729 | 2. 54648 | 9 .26491 | 1.21996 | 6 .24575 | 4.63E-07 | 6.1268 | 0 | 0 | 0/00 | 4 | 3 | 3/1/0 |
| 515.873 | 9. 44347 | 26. 5593 | 3. 64945 | 11. 8849 | 8.65E-10 | 7.93921 | 22 | 3 | 9/6/7 | 29 | 4 | 11/10/8 |
| 537.536 | 378. 672 | 169. 167 | 347. 463 | 63. 4654 | 0.0047706 | -3.06218 | 24 | 3 | 9/9/6 | 21 | 4 | 7/7/7 |
| 613.939 | 43. 4994 | 23. 1673 | 25. 4162 | 9. 18296 | 0.0004051 | -3.9567 | 8 | 1 | 3/2/3 | 11 | 4 | 8/2/1 |
| 616.91 | 19 .4343 | 54. 8513 | 15. 0946 | 63. 2623 | 0.0031649 | 3.15334 | 8 | 3 | 0/6/2 | 14 | 3 | 7/4/3 |
| 622 .257 | 16.8088 | 23. 7614 | 3.40847 | 12.3725 | 0.0032935 | 3.1305 | 3 | 1 | 1/0/2 | 15 | 4 | 7/3/7 |
| 630.856 | 20.6938 | 15. 5097 | 8.14696 | 3.73546 | 0.0042462 | -3.06076 | 9 | 3 | 5/1/3 | 0 | 0 | 0/0/0 |
| 725.273 | 60.78 | 31.6 | 32.1626 | 19.6349 | 0.0001688 | -4.14114 | 24 | 3 | 6/9/9 | 23 | 4 | 7/9/7 |
| 741.318 | 34.4079 | 17. 8617 | 17.2802 | 7.70411 | 5.20E-05 | -4.62387 | 17 | 3 | 3/7/7 | 7 | 3 | 2/4/2 |
| 788.429 | 30.2288 | 20.6407 | 11.4581 | 9.22807 | 0.0009037 | -3.53249 | 17 | 3 | 8/6/3 | 13 | 4 | 5/4/4 |
| 796.39 | 24.214 | 17.448 | 8.95023 | 3.11057 | 0.0007235 | -3.75225 | 13 | 3 | 6/4/3 | 13 | 4 | 5/4/4 |
| 825.062 | 99.2515 | 34.2368 | 85.5689 | 40.6163 | 0.0008758 | -3.6361 | 23 | 3 | 8/6/9 | 21 | 4 | 8/6/7 |
| 842.46 | 911.88 | 452.619 | 506.004 | 236.448 | 0.0001111 | -4.35376 | 27 | 3 | 8/6/9 | 34 | 4 | 12/11/11 |
| 864.614 | 86.9617 | 42.5941 | 49.9207 | 23.112 | 0.0001435 | -4.26908 | 27 | 3 | 9/9/9 | 32 | 4 | 11/10/11 |
| 901.35 | 19.2208 | 13 .1052 | 8.45677 | 2.68185 | 0.0010604 | -3.62587 | 10 | 3 | 5/3/2 | 1 | 1 | 0/1/0 |
| 1045.54 | 227.097 | 83.5326 | 129.477 | 130.931 | 7.35E-05 | -4.28007 | 27 | 3 | 9/9/9 | 34 | 4 | 12/11/11 |
| 1184.75 | 8.38233 | 10.3135 | 1.44016 | 3.11185 | 0.0023415 | 3.21142 | 0 | 0 | 0/0/0 | 4 | 1 | 1/2/1 |
| 1248.67 | 7.43521 | 9.84574 | 2.06567 | 4.25875 | 0.0055534 | 2.83884 | 0 | 0 | 0/0/0 | 5 | 1 | 1/2/2 |
| 1269.76 | 8.05761 | 9.74981 | 1.76648 | 2.90095 | 0.0068571 | 2.60831 | 0 | 0 | 0/0/0 | 4 | 2 | 1/0/3 |
| 1274.68 | 20.963 | 43.8466 | 9.21995 | 36.7108 | 0.0012068 | 3.49843 | 19 | 3 | 6/8/6 | 26 | 4 | 9/8/9 |
| 1277.73 | 15.6697 | 20.5643 | 5.53301 | 8.40005 | 0.008352 | 2.73224 | 18 | 3 | 7/6/5 | 22 | 4 | 7/6/9 |
| 1282.72 | 7.72289 | 10.8695 | 1.30683 | 5.53924 | 0.0027782 | 3.20201 | 0 | 0 | 0/0/0 | 7 | 1 | 2/3/2 |
| 1290.78 | 8.46801 | 11.2979 | 1.74642 | 5.38637 | 0.0062903 | 2.87885 | 0 | 0 | 0/0/0 | 8 | 3 | 2/4/2 |
| 1325.76 | 22.6535 | 15.2906 | 10.4958 | 9.84918 | 0.0071382 | -2.79629 | 26 | 3 | 8/9/9 | 11 | 3 | 4/3/4 |
| 1396.81 | 7.30824 | 9.18133 | 1.28187 | 3.69646 | 0.0086366 | 2.75355 | 0 | 0 | 0/0/0 | 9 | 2 | 2/3/4 |
| 1442.81 | 12.8313 | 8.8864 | 3.54718 | 3.30942 | 4.43E-05 | -4.4436 | 18 | 3 | 2/8/8 | 3 | 2 | 1/1/1 |
| 1466.82 | 11. 0005 | 8.2812 | 3.53704 | 3.0265 | 0.0025214 | -3.17666 | 5 | 2 | 0/1/4 | 1 | 1 | 0/1/0 |
| 1505.81 | 7.23864 | 14.6642 | 1.11111 | 14.4531 | 0.005227 | 2.9847 | 0 | 0 | 0/0/0 | 9 | 1 | 3/3/3 |
| 1566.8 | 7.91821 | 13.4852 | 2.10523 | 10.3143 | 0.0040552 | 3.0677 | 2 | 1 | 0/0/2 | 9 | 2 | 3/3/3 |
| 1833.97 | 11.2337 | 22.6854 | 4.75023 | 13.8808 | 5.40E-05 | 4.49079 | 9 | 3 | 3/3/3 | 27 | 4 | 8/11/8 |
| 1910.99 | 7.03505 | 8.44743 | 1.40697 | 2.34379 | 0.0051382 | 2.91422 | 0 | 0 | 0/0/0 | 7 | 1 | 2/3/2 |
| 1922.95 | 10.9151 | 15.7314 | 3.79846 | 6.30823 | 0.0005154 | 3.68876 | 11 | 3 | 2/4/5 | 29 | 4 | 9/10/10 |
| 1925.97 | 10.7102 | 13.9789 | 3.7766 | 4.95078 | 0.0033838 | 3.05683 | 6 | 3 | 1/0/5 | 24 | 1 | 8/10/6 |
| 1969.01 | 7.37333 | 11.0774 | 2.32176 | 3.34897 | 4.04E-06 | 5.09017 | 0 | 0 | 0/0/0 | 18 | 3 | 7/6/5 |
| 1987.02 | 10.8938 | 17.3113 | 4.40396 | 8.20355 | 0.0002686 | 3.90726 | 7 | 2 | 2/1/4 | 24 | 4 | 8/10/6 |
| 1989.07 | 9.38798 | 13.4327 | 3.11536 | 6.03838 | 0.0013886 | 3.38018 | 0 | 0 | 0/0/0 | 8 | 2 | 1/3/4 |
| 1993.03 | 6.25783 | 7.71587 | 1.15045 | 2.03977 | 0.0008823 | 3.52188 | 0 | 0 | 0/0/0 | 10 | 2 | 1/3/4 |
| 2003.01 | 6.90726 | 10.1168 | 1.30656 | 3.52314 | 1.34E-05 | 4.90422 | 0 | 0 | 0/0/0 | 7 | 2 | 4/2/1 |
| 2006.01 | 7.11286 | 11.6075 | 1.48333 | 6.64755 | 0.000486 | 3.82439 | 2 | 2 | 0/0/2 | 13 | 3 | 5/5/3 |
| 2044.01 | 7.25269 | 9.7212 | 1.53884 | 3.50696 | 0.0005917 | 3.68215 | 0 | 0 | 0/0/0 | 9 | 1 | 3/3/3 |
| 2065.08 | 6.82965 | 8.06093 | 1.25075 | 2.222014 | 0.0084678 | 2.7334 | 0 | 0 | 0/0/0 | 9 | 1 | 3/3/3 |
| 2121.04 | 10.4431 | 7.72746 | 2.68567 | 2.24851 | 0.0001042 | -4.21115 | 7 | 3 | 1/4/2 | 0 | 0 | 0/0/0 |
| 2167.13 | 14.3241 | 9.78683 | 6.22877 | 3.63731 | 0.0017435 | 3.35765 | 7 | 3 | 2/4/1 | 3 | 2 | 2/0/1 |
| 2195.14 | 24.3767 | 12.6862 | 8.19156 | 5.65711 | 1.12E-07 | -6.31546 | 24 | 3 | 8/9/7 | 20 | 3 | 7/6/7 |
| 2225 .14 | 229.975 | 134.612 | 109. 304 | 109.152 | 0.0013014 | -3.38666 | 27 | 3 | 9/9/9 | 29 | 4 | 11/9/9 |
| 2233.17 | 167. 656 | 46.9804 | 86.3481 | 21.0183 | 9.23E-08 | -7.09684 | 27 | 3 | 9/9/9 | 23 | 3 | 8/7/8 |
| 2246.17 | 16.4594 | 25.1637 | 7.02665 | 16.9516 | 0.0093021 | 2.71475 | 20 | 3 | 8/7/5 | 25 | 4 | 7/10/8 |
| 2255.14 | 42.1485 | 17.7824 | 19.5739 | 7.22485 | 7.49E-07 | -6.14443 | 21 | 3 | 6/8/7 | 13 | 4 | 4/5/4 |
| 2269.2 | 26.3369 | 16.6138 | 10.0547 | 6.668881 | 8.18E-05 | -4.349 | 7 | 3 | 4/3/0 | 8 | 2 | 2/3/3 |
| 2277.12 | 24.6783 | 13.1334 | 11.4348 | 4.47791 | 2.24E-05 | -4.95064 | 7 | 3 | 3/1/3 | 3 | 2 | 2/0/1 |
| 2283.19 | 149.657 | 34.7215 | 76.3971 | 26.0205 | 1.58E-08 | -7.5711 | 27 | 3 | 9/9/9 | 31 | 4 | 11/11/9 |
| 2297.2 | 82.3103 | 62.4811 | 46.6451 | 45.2308 | 0.0014327 | -3.35714 | 27 | 3 | 9/9/9 | 27 | 4 | 9/8/10 |
| 2300.27 | 88.6015 | 60.1078 | 42.9161 | 29.2708 | 0.0002519 | -3.98275 | 20 | 3 | 5/8/7 | 16 | 3 | 5/6/5 |
| 2305.2 | 38.2403 | 12.3083 | 18.0571 | 4.25516 | 5.59E-08 | -7.30299 | 26 | 3 | 9/9/8 | 5 | 2 | 3/2/0 |
| 2319.23 | 23.4308 | 13.0863 | 10.4184 | 4.97491 | 3.36E-05 | -4.74734 | 22 | 3 | 6/8/7 | 10 | 3 | 4/3/3 |
| 2419.32 | 9.552 | 14.9041 | 2.79127 | 5.01204 | 2.41E-06 | 6.28028 | 9 | 3 | 3/5/1 | 23 | 4 | 8/8/7 |
| 2678.33 | 9.44347 | 26.5593 | 3.64945 | 11.8849 | 8.65E-10 | 7.93921 | 13 | 3 | 5/5/3 | 6 | 3 | 1/1/4 |
| 2691.3 | 6.24636 | 36.9592 | 1.39736 | 27.8121 | 2.67E-07 | 6.4289 | 0 | 0 | 0/0/0 | 24 | 3 | 8/8/8 |
| 2706.3 | 9.28678 | 14.714 | 3.50576 | 6.01543 | 0.0008982 | 3.54426 | 6 | 3 | 0/2/4 | 9 | 3 | 2/4/3 |
| 2748.32 | 9.08826 | 28.9299 | 2.88675 | 21.1841 | 4.98E-06 | 5.3987 | 14 | 3 | 2/4/8 | 24 | 3 | 8/8/8 |
| 2766.06 | 9.14353 | 18.7584 | 2.21578 | 8.5206 | 1.99E-07 | 6.31633 | 2 | 2 | 0/1/1 | 3 | 1 | 1/0/2 |
| 2780.41 | 12.8917 | 46.8548 | 8.41174 | 38.3278 | 1.34E-05 | 5.01702 | 11 | 3 | 3/8/0 | 29 | 4 | 10/6/13 |
| 2792.76 | 15.9486 | 24.607 | 5.21157 | 12.0516 | 0.0004566 | 3.76888 | 13 | 3 | 1/6/6 | 5 | 2 | 1/2/2 |
| 2839.36 | 8.00334 | 11.4132 | 2.66019 | 4.62892 | 0.0006691 | 3.60983 | 3 | 1 | 2/1/0 | 7 | 2 | 3/3/1 |
| 2921.35 | 9.81981 | 24.5824 | 3.0698 | 25.0492 | 0.0017062 | 3.40439 | 16 | 3 | 5/5/8 | 20 | 3 | 8/7/5 |
| 2969.46 | 4.28688 | 9.68306 | 0.991791 | 6.60686 | 4.03E-05 | 4.69628 | 0 | 0 | 0/0/0 | 4 | 2 | 1/0/3 |
| 3076.56 | 8.94529 | 17.2518 | 5.03761 | 7.93412 | 6.62E-06 | 4.97174 | 14 | 3 | 6/5/3 | 23 | 3 | 8/8/7 |
| 3094.61 | 7.90327 | 16.9166 | 3.56821 | 5.65689 | 3.61E-10 | 7.5832 | 10 | 3 | 4/4/2 | 20 | 3 | 8/7/5 |
| 3223.15 | 6.11531 | 7.73976 | 1.2673 | 2.02518 | 0.0003274 | 3.82769 | 5 | 3 | 1/2/2 | 2 | 1 | 0/1/1 |
| 3346.68 | 16.7817 | 25.311 | 7.94749 | 11.7148 | 0.0013132 | 3.37793 | 14 | 3 | 6/5/3 | 26 | 4 | 9/8/8 |
| 3353.74 | 17. 1876 | 26.6098 | 8.78887 | 12.3616 | 0.0009674 | 3.47591 | 4 | 2 | 2/1/1 | 7 | 3 | 1/1/5 |
| 3393.64 | 6.1185 | 10.3087 | 2.30376 | 4.05225 | 4.78E-06 | 6.08309 | 0 | 0 | 0/0/0 | 8 | 2 | 2/3/3 |
| 3493.82 | 4.12957 | 6.52261 | 1.42734 | 2.23361 | 4.45E-06 | 5.07675 | 0 | 0 | 0/0/0 | 14 | 2 | 4/5/5 |
| 3651.9 | 4.09495 | 6.62707 | 1.79813 | 2.85198 | 6.78E-05 | 4.2262 | 0 | 0 | 0/0/0 | 10 | 3 | 3/4/3 |
| 3795.87 | 14.9262 | 31.9072 | 9.1474 | 22.0212 | 0.0001793 | 4.07537 | 9 | 2 | | 8 | 2 | 2/3/3 |
| 3809.85 | 17.1109 | 42.4032 | 11.9236 | 29.938 | 4.77E-05 | 4.49737 | 10 | 2 | 1/3/6 | 13 | 4 | 3/5/5 |
| 3970.95 | 2.56797 | 4.16099 | 1.10994 | 1.76872 | 7.00E-05 | 4.29388 | 0 | 0 | 0/0/0 | 2 | 1 | 0/0/2 |
| 4263.15 | 2.60469 | 4.63088 | 1.28554 | 1.54405 | 1.72E-06 | 5.31524 | 0 | 0 | 0/0/0 | 2 | 1 | 1/0/1 |

| | | | | | | | | | | | | |
|---|---|---|---|---|---|---|---|---|---|---|---|---|
| Ave= average; ¹⁾ = number of peptides observed in all spectra; ²⁾ number of patients or controls that contributed; subsamples in x/x/x = measured in the first, the second and the third measurement. | | | | | | | | | | | | |

**Table 5. Differences in peptides of stroma from placentas from early onset preeclampsia (n=4) and preterm delivery (n=3).**

| Ave mass | Ave case | Ave control | SD case | SD control | p-value | t-test | Spectra control ^{1 )} | Controls ²⁾ | Subsamples control | Spectra cases ¹⁾ | Patient cases ²⁾ | Subsampl es cases |
|---|---|---|---|---|---|---|---|---|---|---|---|---|
| 262.988 | 13.8738 | 2.71653 | 10.7176 | 1.15289 | 1.71E-06 | 5.84894 | 0 | 0 | 0/0/0 | 10 | 3 | 4/3/3 |
| 590.024 | 61.2175 | 41.2819 | 34.2643 | 22.5479 | 0.0098543 | 2.67554 | 7 | 3 | 2/4/1 | 8 | 3 | 2/4/2 |
| 614.051 | 25.1373 | 47.0355 | 9.44988 | 39.1856 | 0.0083272 | -2.83506 | 20 | 3 | 8/7/3 | 7 | 4 | 5/2/0 |
| 620.296 | 31.9927 | 19.2892 | 16.6779 | 11.6128 | 0.0011367 | 3.43376 | 3 | 2 | 2/1/0 | 13 | 4 | 7/3/3 |
| 656.023 | 154.434 | 44.7987 | 13.774 | 35.0254 | 0.0001344 | 4.28068 | 24 | 3 | 9/6/9 | 32 | 4 | 11/11/10 |
| 659.167 | 36.4743 | 20.1289 | 16.5093 | 10.1047 | 2.12E-05 | 4.6608 | 4 | 2 | 2/2/0 | 14 | 3 | 4/6/4 |
| 665.986 | 55.5578 | 31.0713 | 42.3998 | 20.2903 | 0.0057428 | 2.89731 | 10 | 3 | 3/4/3 | 20 | 4 | 5/8/7 |
| 672.304 | 26.2096 | 17.3044 | 12.6069 | 6.54895 | 0.0010815 | 3.47817 | 0 | 0 | 0/0/0 | 4 | 3 | 1/2/1 |
| 681.122 | 103. 104 | 32.7945 | 71.5925 | 15.777 | 4.93E-06 | 5.40218 | 19 | 3 | 8/4/7 | 31 | 4 | 11/10/9 |
| 706.05 | 61.8267 | 25.7221 | 35.7525 | 10.0457 | 3.44E-06 | 5.46272 | 13 | 3 | 8/2/3 | 29 | 4 | 10/10/9 |
| 713.813 | 25.6543 | 19.5254 | 10.5602 | 5.67433 | 0.0066606 | 2.83386 | 4 | 3 | 4/0/0 | 6 | 2 | 2/2/2 |
| 731. 125 | 59.2565 | 37.6978 | 26.1338 | 20.8652 | 0.0008521 | 3.52206 | 19 | 3 | 8/6/3 | 28 | 4 | 10/9/9 |
| 741.223 | 186731 | 37.7905 | 7.80142 | 25.1625 | 0.0006595 | -3.79684 | 15 | 3 | 5/3/7 | 3 | 2 | 1/1/1 |
| 776.333 | 20.9718 | 27.6288 | 6.0691 | 8.64863 | 0.0015776 | -3.3617 | 4 | 2 | 4/0/0 | 1 | 1 | 0/2/0 |
| 796.324 | 19.0124 | 22.853 | 3.73905 | 6.1466 | 0.0070715 | -2.83425 | 7 | 2 | 1/4/2 | 0 | 0 | 0/0/0 |
| 850.075 | 32.8469 | 18.0857 | 15.7007 | 8.54301 | 3.22E-05 | 4.5758 | 7 | 3 | 3/3/1 | 25 | 4 | 9/8/8 |
| 856.6 | 33.3832 | 20.4727 | 17.349 | 8.8306 | 0.0005897 | 3.65212 | 10 | 3 | 1/7/2 | 22 | 4 | 8/7/7 |
| 861.118 | 28.1182 | 20.055 | 11.5713 | 9.27223 | 0.0043501 | 2.97045 | 9 | 3 | 1/7/2 | 24 | 4 | 9/8/7 |
| 867.279 | 21.6574 | 13.013 | 12.5614 | 2.57365 | 0.0005718 | 3.79956 | 0 | 0 | 0/0/0 | 9 | 2 | 2/4/3 |
| 870.586 | 45.1729 | 29.866 | 22.3439 | 14.947 | 0.0027899 | 3.13259 | 21 | 3 | 7/8/6 | 32 | 4 | 12/11/9 |
| 877.092 | 32.0511 | 15.8613 | 24.0935 | 8.23402 | 0.0009815 | 3.56257 | 4 | 3 | 2/2/0 | 18 | 4 | 5/7/6 |
| 886.333 | 18.9041 | 13.8576 | 8.52964 | 3.77335 | 0.0042417 | 3.0154 | 1 | 1 | 0/0/1 | 5 | 2 | 2/2/1 |
| 892.292 | 20.971 | 11.4263 | 12.1759 | 2.54343 | 0.0001257 | 4.32401 | 0 | 0 | 0/0/0 | 9 | 3 | 3/4/2 |
| 896. 302 | 17.1758 | 12.1348 | 6.14244 | 2.62382 | 0.0001263 | 4.20961 | 0 | 0 | 0/0/0 | 3 | 2 | 2/1/0 |
| 1004.6 | 17.8569 | 9.70526 | 14.7818 | 1.39701 | 0.0040114 | 3.10318 | 0 | 0 | 0/0/0 | 30 | 1 | 2/3/3 |
| 1045.59 | 62.1096 | 162.651 | 44.8452 | 107.291 | 6.75E-05 | -4.5474 | 26 | 3 | 8/9/8 | 30 | 4 | 10/10/10 |
| 1282.63 | 10.1684 | 7.47772 | 3.3926 | 1.04673 | 0.0001333 | 4.25296 | 0 | 0 | 0/0/0 | 6 | 1 | 0/3/3 |
| 1247.85 | 9.95604 | 13.3618 | 3.33148 | 5.42244 | 0.0069078 | -2.84225 | 11 | 3 | 2/4/5 | 2 | 2 | 0/1/1 |
| 1442.75 | 7.63388 | 12.3484 | 3.00514 | 3.74956 | 3.05E-06 | -5.26141 | 9 | 2 | 1/5/3 | 0 | 0 | 0/0/0 |
| 1505.83 | 15.9917 | 7.28597 | 15.2648 | 1.45867 | 0.0030471 | 3.20885 | 9 | 1 | 3/3/3 | 0 | 0 | 0/0/0 |
| 1567.8 | 11.6434 | 7.17592 | 7.99041 | 1.60837 | 0.0039846 | 3.08944 | 9 | 1 | 3/3/3 | 0 | 0 | 0/0/0 |
| 1621.89 | 7.31068 | 10.5596 | 2.2698 | 3.85692 | 0.0004107 | -3.85041 | 6 | 2 | 1/2/3 | 1 | 1 | 0/1/0 |
| 1774.89 | 7.15505 | 9.43972 | 2.7908 | 2.39548 | 0.0012998 | -3.3836 | 3 | 2 | 0/1/2 | 0 | 0 | 0/0/0 |
| 1833.97 | 23.3924 | 12.94 | 11.2512 | 6.68514 | 5.22E-05 | 4.41255 | 11 | 3 | 3/2/6 | 17 | 4 | 6/7/4 |
| 1912.08 | 8.64263 | 6.98318 | 2.85834 | 1.50498 | 0.0064143 | 2.84927 | 0 | 0 | 0/0/0 | 5 | 1 | 0/3/2 |
| 1922.97 | 16.0702 | 11.9959 | 6.23042 | 4.46409 | 0.0050903 | 2.91685 | 11 | 3 | 4/3/4 | 25 | 4 | 8/9/7 |
| 1925.01 | 14.1119 | 11.0599 | 4.89031 | 3.79585 | 0.0092116 | 2.69649 | 3 | 2 | 0/2/1 | 3 | 3 | 0/2/1 |
| 1969.01 | 10.2268 | 7.99624 | 2.9029 | 2.67845 | 0.0033183 | 3.06668 | 0 | 0 | 0/0/0 | 10 | 2 | 4/4/2 |
| 1987.01 | 19.0747 | 11.1726 | 11.8143 | 4.73492 | 00012222 | 3.46788 | 8 | 1 | 2/3/3 | 15 | 3 | 5/4/6 |
| 2002.99 | 10.9827 | 7.22838 | 5.42922 | 1.97252 | 0.000863 | 3.60135 | 6 | 2 | 0/0/6 | 9 | 2 | 3/3/2 |
| 2009.04 | 9.62746 | 7.92852 | 2.40804 | 2.04619 | 0.0048719 | 2.92974 | 0 | 0 | 0/0/0 | 8 | 1 | 3/2/3 |
| 2121.02 | 7.42291 | 10.8348 | 2.18169 | 2.71602 | 3.14E-06 | -5.25238 | 6 | 3 | 0/4/2 | 0 | 0 | 0/0/0 |
| 2167.17 | 8.49596 | 14.0443 | 2.96242 | 5.32158 | 2.17E-05 | -4.82356 | 12 | 3 | 3/5/4 | 0 | 0 | 0/0/0 |
| 2195.16 | 11.3508 | 22.3006 | 4.78999 | 8.99456 | 1.54E-06 | -5.68224 | 24 | 3 | 9/8/7 | 8 | 3 | 2/4/2 |
| 2225.11 | 101.838 | 194.542 | 72.3586 | 99.1678 | 0.0002009 | -4.03502 | 25 | 3 | 9/7/9 | 23 | 3 | 8/8/7 |
| 2229.25 | 21.0833 | 34.21 | 11.9151 | 15.9005 | 0.0009247 | -3.5335 | 26 | 3 | 9/8/8 | 30 | 4 | 9/11/10 |
| 2233.27 | 43.223 | 131.011 | 17.5229 | 86.3174 | 1.66E-05 | -5.19514 | 26 | 3 | 9/8/9 | 22 | 3 | 8/8/6 |
| 2255.27 | 16.7614 | 34.234 | 7.18119 | 19.6326 | 0.0001188 | -4.38364 | 23 | 3 | 8/8/7 | 6 | 3 | 3/1/2 |
| 2269.3 | 14.9051 | 24.0354 | 5.71852 | 12.022 | 0.0009136 | -3.61617 | 19 | 3 | 5/7/7 | 13 | 3 | 5/6/2 |
| 2277.19 | 11.812 | 21.7456 | 4.6842 | 10.5847 | 7.00E-05 | -4.51758 | 14 | 3 | 4/5/5 | 4 | 1 | 2/2/0 |
| 2283.24 | 26.8137 | 144.125 | 15.6262 | 80.565 | 4.52E-08 | -7.44249 | 26 | 3 | 9/8/9 | 28 | 4 | 8/10/10 |
| 2297.37 | 39.8225 | 96.8226 | 28.4447 | 54.8085 | 1.99E-05 | -4.878 | 25 | 3 | 9/8/8 | 30 | 4 | 9/11/10 |
| 2300.33 | 36.4582 | 85.3599 | 18.7976 | 48.0605 | 2.03E-05 | -4.97572 | 17 | 3 | 7/7/3 | 8 | 3 | 2/2/4 |
| 2305. 17 | 11.2787 | 34.437 | 4.34527 | 19.0142 | 1.04E-06 | -6.19366 | 23 | 3 | 8/8/7 | 1 | 1 | 0/0/1 |
| 2319.29 | 13.3122 | 24.2634 | 5.48272 | 12.8219 | 0.0002226 | -4.1308 | 18 | 3 | 8/6/4 | 5 | 2 | 2/2/1 |
| 2419. 31 | 16.4153 | 11.3734 | 5.09731 | 4.78704 | 0.0027181 I81 | 3.13646 | 9 | 2 | 2/4/3 | 17 | 4 | 7/5/5 |
| 2678.36 | 22.4772 | 10.4965 | 8.23497 | 4.66681 | 5.69E-09 | 7.0042 | 5 | 2 | 3/1/1 | 18 | 4 | 4/8/6 |
| 2691.33 | 28.6942 | 9.76988 | 20.2647 | 3.2826 | 1.02E-05 | 5.20241 | 0 | 0 | 0/0/0 | 21 | 3 | 3/6/6 |
| 2748.33 | 22.3689 | 10.5837 | 16.0865 | 4.5916 | 0.0003317 | 3.95762 | 13 | 3 | 2/5/6 | 20 | 3 | 9/5/6 |
| 2808.42 | 95.0629 | 187.118 | 82.2599 | 153.602 | 0.008087 | -2.7943 | 25 | 3 | 8/8/9 | 24 | 3 | 9/8/7 |
| 2969.55 | 13.9863 | 5.86417 | 9.55017 | 1.84316 | 4.16E-05 | 4.70817 | 10 | 2 | 4/2/4 | 0 | 0 | 0/0/0 |
| 3094.68 | 18.1253 | 9.28129 | 8.22074 | 4.93244 | 4.96E-06 | 5.09509 | 8 | 3 | 3/3/2 | 14 | 3 | 7/2/5 |
| 3353.81 | 23.7099 | 16.6219 | 8.68048 | 9.25527 | 0.0039127 | 3.0184 | 3 | 2 | 1/2/0 | 17 | 3 | 5/7/5 |
| 3392.82 | 8.82184 | 6.04125 | 3.31654 | 2.25673 | 0.0003473 | 3.81597 | 0 | 0 | 0/0/0 | 7 | 1 | 3/1/3 |
| 3510.83 | 6.38991 | 3.92034 | 2.48897 | 0.98284 | 6.46E-06 | 5.1564 | 0 | 0 | 0/0/0 | 6 | 2 | 2/1/3 |
| 3651.99 | 6.42466 | 4.33235 | 2.10185 | 1.35311 | 2.53E-05 | 4.61142 | 3 | 2 | 3/3/2 | 10 | 3 | 4/3/3 |
| 3957.03 | 5.27971 | 3.26215 | 1.80184 | 1.05737 | 2.16E-06 | 5.33779 | 0 | 0 | 0/0/0 | 8 | 2 | 4/3/3 |
| 3972.03 | 6.70117 | 2.92008 | 3.90731 | 1.01906 | 6.69E-06 | 6.26596 | 0 | 0 | 0/0/0 | 10 | 2 | 2/3/3 |

| | | | | | | | | | | | | |
|---|---|---|---|---|---|---|---|---|---|---|---|---|
| Ave= average; ¹⁾ = number of peptides observed in all spectra; ²⁾ number of patients or controls that contributed; subsamples in x/x/x = measured in the first, the second and the third measurement. | | | | | | | | | | | | |

### 1.2.3. Off-line NanoLC MALDI measurements

Using the Ultimate NanoLC system and MALDI TOF/TOF measurements resulted in the identification of four masses of differentially expressed proteins that were confirmed by FT-MS (a tolerance of 4 ppm was allowed using external calibration). Subsequently, we focused on proteins specific and discriminating for early onset preeclampsia only (neither detectable in controls nor late onset preeclampsia). Significantly differentially expressed proteins include choriomammotropin A precursor (LCHUC), fragment OTTHUM (Q5T6S7), surfeit locus protein 4 (015260) and calcyclin (P06703) (Table 6).

**Table 6. Identified and confirmed significantly differentially expressed proteins in early onset preeclampsia placentas.**

| Ave Mass MALDI-Tof | Calc. mass | [prot_desc] | Score | Matched peptides | MW | FTMS | ppm |
|---|---|---|---|---|---|---|---|
| 1269.760 | 1269.6194 | choriomammotropin A precursor [validated] (LCHUC) | 66 | 4 | 25004 | 1269.6142 | 4 |
| 1910.990 | 1910.7899 | OTTHUMP000000 18488 (Fragment) (Q5T6S7_HUMAN) | 20 | 3 | 28076 | 1910.7928 | 1.6 |
| 2044.010 | 2043.9127 | Surfeit locus protein 4 (O15260) | 67 | 2 | 30374 | 2043.9056 | 3.5 |
| 891.410 | 891.4240 | Calcyclin (Prolactin receptor associated protein) (PRA) | 51 | 2 | 10173 | 891.4233 | 0.8 |
| 915.560 | 915.4894 | (Growth factor-inducible protein 2 (P06703)) | | | | 915.4898 | 0.4 |

Calcyclin, was verified by immunostaining, for the other proteins no commercial antibodies were available yet. Immunohistochemistry showed for calcyclin positive staining for trophoblasts from placenta of women with early onset preeclampsia compared with controls (Fig. 7). Table 7 represents the expression levels of calcyclin in the early onset preeclampsia, control and preterm group. By immunohistochemistry, it was evident that choriomammotropin (using an antibody that is not specific for the precursor) is present both in trophoblast of preeclampsia and control placental tissue.

**Table 7. The contents of the S100A6 (calcyclin) in the early onset preeclampsia, control and preterm group performed by immunohistochemical staining.**

| no. | study samples | expression level (S100A6) in trophoblast |
|---|---|---|
| 1 | early onset PE | ++ |
| 2 | early onset PE | + |
| 3 | early onset PE | + |
| 4 | control | - |
| 5 | control | +/- |
| 6 | control | +/- |
| 7 | preterm control | - |
| 8 | preterm control | - |

| | | |
|---|---|---|
| Samples used for immunohistochemistry were not taken from the test cohort used in the mass spectrometry experiments. - no staining in trophoblasts; +/- faint cytoplasma staining in trophoblasts; + and ++ strong staining in trophoblast cells. | | |

### 1.3. Conclusion

This Example describes different tryptic peptide patterns of villous trophoblast and stroma cells from placentas of women with pregnancies complicated by preeclampsia (cases) compared to uncomplicated pregnancies (controls) in a small number of 125 microdissected cells. We subsequently found that these peptide patterns are different in placentas from early onset preeclampsia (< 34 weeks gestation) compared to a late onset of the disease (> 34 weeks gestation. In order to determine whether this is an effect of preterm delivery per se, we compared the peptide patterns of women with early onset preeclampsia with women who had a pregnancy complicated by preterm delivery of unknown cause. These data confirmed that the peptides patterns are specific for early onset preeclampsia (as compared to late onset preeclampsia as well as controls) and are not the effect of preterm delivery. We identified as much as possible proteins in a representative tissue section of the three groups (preeclampsia, controls, preterm deliveries) by MSMS sequencing and confirmed the identification by accurate mass FT ICR MS measurement. Four proteins correspond to the differentially expressed peptides by accurate FT-MS measurement. By immunohistochemistry we could confirm that calcyclin is significantly different (black white difference) in expression. We used 8 paraffine sections of the early onset preeclampsia (n=3), control (n=3) and preterm (n=2) group for the immunohistochemical staining.

Calcyclin, S100A6, is a member of the S-100 calcium-binding protein family (12). Recently, it has been described that calcyclin stimulates secretion of choriomammotropin (placental lactogen). A dose-dependent release in isolated mice trophoblasts of placental lactogen in response to calcyclin was described by independent researchers. Uterine natural killer (NK) cells were the primary site of calcyclin RNA messengers and calcyclin is expressed in natural killer cells throughout pregnancy. Uterine natural killer cells have been implicated in trophoblast function. The present finding is further supported by the reported reduced natural killer cell proportion primarily in pregnancies complicated by impaired placental development (trophoblast infiltration and placental growth) such as preeclampsia. Furthermore, calcyclin secretion is further controlled by cytokines such as interleukin-1. Interleukin-1 is spontaneously synthesized during preeclampsia. This implies a specific and different mechanism in early onset preeclampsia through calcyclin and choriomammotropin. Although, we observed by imunohistochemistry that choriomammatropin is present in both the preeclamptic and control trophoblasts, the differences in the expression of the choriomammatropin precursor as judged by our proteomics experiment suggests some involvement in the pathophysiology of this metabolic pathway.

Surfeit locus protein 4 is another peptide which is differentially expressed in placentas from women with early onset preeclampsia. The surfeit locus genes contain a tight cluster of mammalian genes. The surfeit locus 4 gene is encoding an integral membrane protein most likely associated with the endoplasmic reticulum. No association with placentation or human development has hitherto been described.

Recently, it was reported that elevated clusterin levels could be found in serum from women with preeclampsia. Clusterin was identified as over expressed spots on two-dimensional electrophoresis with MALDI-TOF-MS followed by Western blot analysis. Using placenta samples in line with the hypothesis that the responsible trigger for the disease is mostly likely located within the placenta, we could not confirm that clusterin is involved in the pathophysiology of preeclampsia.

Overall, early onset preeclampsia results more often in severe maternal and fetal complications although it is not an all or nothing phenomenon. Recent longitudinal studies of placental growth by ultrasound have revealed that in cases of early onset preeclampsia the placenta volume is reduced from as early as 12 weeks of gestation. Calcyclin has been detected also in early pregnancy during implantation in mice. Moreover, in the first trimester the endovascular trophoblast invasion - and subsequent conversion of the spiral arteries and release of the endovascular trophoblastic plugs allowing intervillous blood flow- is disturbed in early onset preeclampsia. By contrast, in cases of late onset preeclampsia, placental volume is slightly larger than normal at 12 weeks gestation and the placental insult seems less clear as villous growth is normal. This suggests a different underlying mechanism of early and late onset preeclampsia. In addition, we firstly found specific peptides in early onset preeclampsia in trophoblasts that were striking different from stroma cells. This suggests that abnormal placentation is not due to disturbed trophoblast metabolism only but that an abnormal interaction between villous trophoblast and villous stroma cells may be involved as well. In the near future, developments in clinical proteomics in terms of sensitivity, reproducibility, resolution and accuracy will help us to use this method for the analysis of small numbers of trophoblasts and stroma cells obtained in early pregnancy to predict subsequent preeclampsia in the second half of pregnancy.

In conclusion, in the present Example, specific peptides of placentas from women with pregnancies complicated by early onset preeclampsia are described. Specific peptide patterns were found, using small numbers of cells, in villous trophoblast and their corresponding proteins could be identified being choriomammotropin precursor, calcyclin and surfeit locus protein 4.

## Claims

1. A method for detecting early-onset preeclampsia in a subject, comprising determining the expression level of the protein calcyclin in a sample of chorionic villi of said subject.

2. Method according to claim 1, wherein said expression level is determined at a point in time between 10 and 34 weeks gestation, preferably between 10 and 20 weeks gestation, more preferably between 10 and 15 weeks gestation.

3. Method according to claim 1 or 2, wherein said expression level is determined in stromal cells or trophoblast cells, most preferably trophoblasts.

4. Method according to any one of the preceding claims, wherein in addition to said expression level of calcyclin, also the expression level of one or more of the proteins selected from surfeit locus protein, human protein OTTHUMP00000018488 having the amino acid sequence of SEQ ID NO: 4 and choriomammotropin A precursor is determined.

5. Method according to any one of the preceding claims, wherein said expression level is determined by detecting the said protein or a peptide fragment thereof in a mass range of 800 to 27,000 Da.

6. Method according to any one of the preceding claims, wherein said detection is performed by immunohistochemical analysis or mass spectrometry.

7. Use of a method according to any one of claims 1-6, wherein said use is for monitoring the disease process of early-onset preeclampsia or a response to a therapy during the disease.

8. Use of a marker protein or marker peptide for detecting early-onset preeclampsia in a subject wherein said marker protein is the expression level of the protein calcyclin in a sample of chorionic villi of said subject and wherein said marker peptide is a peptide fragment of calcyclin in a sample of chorionic villi of said subject having a mass of between 800 and 27,000 Da.

9. Use according to claim 8, wherein said expression level is the expression level at a stage in the pregnancy between week 10 and week 34 gestation.

10. Use of a marker protein as defined in claim 8 or 9, or of a marker profile comprising the expression level in a sample of chorionic villi of a subject of a first protein being calcyclin or a peptide thereof, and wherein said marker profile further comprises at least one additional expression level of a protein or peptide fragment selected from the group of surfeit locus protein, human protein OTTHUMP00000018488 having the amino acid sequence of SEQ ID NO.: 4, and choriomammotropin A precursor, for detecting early-onset preeclampsia in a pregnant woman.

## Patentansprüche

1. Verfahren zum Nachweis von früher Präeklampsie bei einer Patientin, umfassend das Bestimmen des Expressionsniveaus des Proteins Calcyclin in einer Probe von Chorionzotten der Patientin.

2. Verfahren gemäß Anspruch 1, wobei das Expressionsniveau zu einem Zeitpunkt zwischen der 10. und 34. Schwangerschaftswoche, vorzugsweise zwischen der 10. und 20. Schwangerschaftswoche, besonders bevorzugt zwischen der 10. und 15. Schwangerschaftswoche, bestimmt wird.

3. Verfahren gemäß Anspruch 1 oder 2, wobei das Expressionsniveau in Stromazellen oder Trophoblastenzellen, am meisten bevorzugt Trophoblasten, bestimmt wird.

4. Verfahren gemäß einem der vorstehenden Ansprüche, wobei außer dem Expressionsniveau von Calcyclin auch das Expressionsniveau von einem oder mehreren der Proteine bestimmt wird, die aus einem Surfeit-Locus-Protein, dem humanen Protein OTTHUMP00000018488 mit der Aminosäuresequenz von SEQ ID Nr. 4 und dem Choriomammotropin-A-Vorläufer ausgewählt sind.

5. Verfahren gemäß einem der vorstehenden Ansprüche, wobei das Expressionsniveau dadurch bestimmt wird, dass man das Protein oder ein Peptidfragment davon in einem Massenbereich von 800 bis 27000 Da nachweist.

6. Verfahren gemäß einem der vorstehenden Ansprüche, wobei der Nachweis durch immunhistochemische Analyse oder Massenspektrometrie durchgeführt wird.

7. Verwendung eines Verfahrens gemäß einem der Ansprüche 1 bis 6, wobei die Verwendung der Verlaufskontrolle von früher Präeklampsie oder der Kontrolle des Ansprechens auf eine Therapie während der Krankheit dient.

8. Verwendung eines Markerproteins oder Markerpeptids zum Nachweis von früher Präeklampsie bei einer Patientin, wobei es sich bei dem Markerprotein um das Expressionsniveau des Proteins Calcyclin in einer Probe von Chorionzotten der Patientin handelt und wobei das Markerpeptid ein Peptidfragment von Calcyclin in einer Probe von Chorionzotten der Patientin mit einer Masse zwischen 800 und 27 000 Da ist.

9. Verwendung gemäß Anspruch 8, wobei es sich bei dem Expressionsniveau um das Expressionsniveau in dem Schwangerschaftsstadium zwischen der 10. und 34. Schwangerschaftswoche handelt.

10. Verwendung eines Markerproteins gemäß Anspruch 8 oder 9 oder eines das Expressionsniveau umfassenden Markerprofils eines ersten Proteins, bei dem es sich um Calcyclin oder ein Peptid davon handelt, in einer Probe von Chorionzotten einer Patientin, wobei das Markerprofil weiterhin wenigstens ein zusätzliches Expressionsniveau eines Proteins oder Peptidfragments, das aus der Gruppe Surfeit-Locus-Protein, humanes Protein OTTHUMP00000018488 mit der Aminosäuresequenz von SEQ ID Nr. 4 und Choriomammotropin-A-Vorläufer ausgewählt ist, umfasst, zum Nachweis von früher Präeklampsie bei einer Schwangeren.

## Revendications

1. Procédé pour détecter une pré-éclampsie à déclenchement précoce chez un sujet comprenant la détermination du niveau d'expression de la protéine calcycline dans un échantillon de villosités choriales dudit sujet.

2. Procédé selon la revendication 1 où ledit niveau d'expression est déterminé à un point dans le temps entre 10 et 34 semaines de gestation, de préférence entre 10 et 20 semaines de gestation, de préférence encore entre 10 et 15 semaines de gestation.

3. Procédé selon la revendication 1 ou 2 où ledit niveau d'expression est déterminé dans des cellules stromales ou des cellules trophoblastiques, de manière particulièrement préférable dans des trophoblastes.

4. Procédé selon l'une quelconque des revendications précédentes où, en plus dudit niveau d'expression de calcycline, également le niveau d'expression d'une ou plusieurs des protéines choisies parmi la protéine surfeit locus, la protéine humaine OTTHUMP00000018488 ayant la séquence d'aminoacides de SEQ ID NO: 4 et le précurseur d'hormone lactogène placentaire A est déterminé.

5. Procédé selon l'une quelconque des revendications précédentes où ledit niveau d'expression est déterminé par détection de ladite protéine ou d'un fragment peptidique de celle-ci dans une plage de masse de 800 à 27 000 Da.

6. Procédé selon l'une quelconque des revendications précédentes où ladite détection est accomplie par analyse immunohistochimique ou spectrométrie de masse.

7. Utilisation d'un procédé selon l'une quelconque des revendications 1-6 où ladite utilisation est pour suivre le processus morbide de pré-éclampsie à déclenchement précoce ou une réponse à une thérapie pendant la maladie.

8. Utilisation d'une protéine marqueur ou d'un peptide marqueur pour détecter une pré-éclampsie à déclenchement précoce chez un sujet où ladite protéine marqueur est le niveau d'expression de la protéine calcycline dans un échantillon de villosités choriales dudit sujet et où ledit peptide marqueur est un fragment peptidique de calcycline dans un échantillon de villosités choriales dudit sujet ayant une masse entre 800 à 27 000 Da.

9. Utilisation selon la revendication 8 où ledit niveau d'expression est le niveau d'expression à un stade dans la grossesse entre 10 semaines et 34 semaines de gestation.

10. Utilisation d'une protéine marqueur selon la revendication 8 ou 9 ou d'un profil marqueur comprenant le niveau d'expression dans un échantillon de villosités choriales d'un sujet d'une première protéine qui est la calcycline ou un peptide de celle-ci, et où ledit profil marqueur comprend en outre au moins un niveau d'expression supplémentaire d'une protéine ou d'un fragment peptidique choisi dans le groupe de la protéine surfeit locus, de la protéine humaine OTTHUMP00000018488 ayant la séquence d'aminoacides de SEQ ID NO: 4 et du précurseur d'hormone lactogène placentaire A pour détecter une pré-éclampsie à déclenchement précoce chez une femme enceinte.


### SEQUENCE LISTING

<110> Erasmus University Medical Center Rotterdam
<120> Peptide markers for diagnosis of preeclampsia
<130> P83501PC00
<150> PCT/NL2007/050014
   <151> 2007-01-12
<160> 4
<170> PatentIn version 3.3
<210> 1
   <211> 90
   <212> PRT
   <213> Artificial sequence
<220>
   <223> amino acid sequence of human calcyclin
<400> 1
   Met Ala Cys Pro Leu Asp Gln Ala Ile Gly Leu Leu Val Ala Ile Phe
   1               5                   10                  15
   His Lys Tyr Ser Gly Arg Glu Gly Asp Lys His Thr Leu Ser Lys Lys
               20                  25                  30
   Glu Leu Lys Glu Leu Ile Gln Lys Glu Leu Thr Ile Gly Ser Lys Leu
           35                  40                  45
   Gln Asp Ala Glu Ile Ala Arg Leu Met Glu Asp Leu Asp Arg Asn Lys
       50                  55                  60
   Asp Gln Glu Val Asn Phe Gln Glu Tyr Val Thr Phe Leu Gly Ala Leu
   65                  70                  75                  80
   Ala Leu Ile Tyr Asn Glu Ala Leu Lys Gly
                   85                  90
<210> 2
   <211> 269
   <212> PRT
   <213> Artificial sequence
<220>
   <223> amino acid sequence of human surfeit locus protein
<400> 2
   Met Gly Gln Asn Asp Leu Met Gly Thr Ala Glu Asp Phe Ala Asp Gln
   1               5                   10                  15
   Phe Leu Arg Val Thr Lys Gln Tyr Leu Pro His Val Ala Arg Leu Cys
               20                  25                  30
   Leu Ile Ser Thr Phe Leu Glu Asp Gly Ile Arg Met Trp Phe Gln Trp
           35                  40                  45
   Ser Glu Gln Arg Asp Tyr Ile Asp Thr Thr Trp Asn Cys Gly Tyr Leu
       50                  55                  60
   Leu Ala Ser Ser Phe Val Phe Leu Asn Leu Leu Gly Gln Leu Thr Gly
   65                  70                  75                  80
   Cys Val Leu Val Leu Ser Arg Asn Phe Val Gln Tyr Ala Cys Phe Gly
                   85                  90                  95
   Leu Phe Gly Ile Ile Ala Leu Gln Thr Ile Ala Tyr Ser Ile Leu Trp
               100                 105                 110
   Asp Leu Lys Phe Leu Met Arg Asn Leu Ala Leu Gly Gly Gly Leu Leu
           115                 120                 125
   Leu Leu Leu Ala Glu Ser Arg Ser Glu Gly Lys Ser Met Phe Ala Gly
   130 135 140
   Val Pro Thr Met Arg Glu Ser Ser Pro Lys Gln Tyr Met Gln Leu Gly
   145 150 155 160
   Gly Arg Val Leu Leu Val Leu Met Phe Met Thr Leu Leu His Phe Asp
   165 170 175
   Ala Ser Phe Phe Ser Ile Val Gln Asn Ile Val Gly Thr Ala Leu Met
   180 185 190
   Ile Leu Val Ala Ile Gly Phe Lys Thr Lys Leu Ala Ala Leu Thr Leu
   195 200 205
   Val Val Trp Leu Phe Ala Ile Asn Val Tyr Phe Asn Ala Phe Trp Thr
   210 215 220
   Ile Pro Val Tyr Lys Pro Met His Asp Phe Leu Lys Tyr Asp Phe Phe
   225 230 235 240
   Gln Thr Met Ser Val Ile Gly Gly Leu Leu Leu Val Val Ala Leu Gly
   245 250 255
   Pro Gly Gly Val Ser Met Asp Glu Lys Lys Lys Glu Trp
   260 265
<210> 3
   <211> 217
   <212> PRT
   <213> Artificial sequence
<220>
   <223> amino acid sequence of human choriomammotropin A precursor
<400> 3
   Met Ala Pro Gly Ser Arg Thr Ser Leu Leu Leu Ala Phe Ala Leu Leu
   1               5                   10                  15
   Cys Leu Pro Trp Leu Gln Glu Ala Gly Ala Val Gln Thr Val Pro Leu
               20                  25                  30
   Ser Arg Leu Phe Asp His Ala Met Leu Gln Ala His Arg Ala His Gln
           35                  40                  45
   Leu Ala Ile Asp Thr Tyr Gln Glu Phe Glu Glu Thr Tyr Ile Pro Lys
       50                  55                  60
   Asp Gln Lys Tyr Ser Phe Leu His Asp Ser Gln Thr Ser Phe Cys Phe
   65                  70                  75                  80
   Ser Asp Ser Ile Pro Thr Pro Ser Asn Met Glu Glu Thr Gln Gln Lys
                   85                  90                  95
   Ser Asn Leu Glu Leu Leu Arg Ile Ser Leu Leu Leu Ile Glu Ser Trp
               100                 105                 110
   Leu Glu Pro Val Arg Phe Leu Arg Ser Met Phe Ala Asn Asn Leu Val
           115                 120                 125
   Tyr Asp Thr Ser Asp Ser Asp Asp Tyr His Leu Leu Lys Asp Leu Glu
       130                 135                 140
   Glu Gly Ile Gln Thr Leu Met Gly Arg Leu Glu Asp Gly Ser Arg Arg
   145                 150                 155                 160
   Thr Gly Gln Ile Leu Lys Gln Thr Tyr Ser Lys Phe Asp Thr Asn Ser
                   165                 170                 175
   His Asn His Asp Ala Leu Leu Lys Asn Tyr Gly Leu Leu Tyr Cys Phe
               180                 185                 190
   Arg Lys Asp Met Asp Lys Val Glu Thr Phe Leu Arg Met Val Gln Cys
           195                 200                 205
   Arg Ser Val Glu Gly Ser Cys Gly Phe
   210 215
<210> 4
   <211> 276
   <212> PRT
   <213> Artificial sequence
<220>
   <223> amino acid sequence of human protein OTTHUMP00000018488
<400> 4
   Val Val Glu Pro Lys Arg Ala Val Ser Arg Glu Asp Ser Val Lys Pro
   1 5 10 15
   Val Ala His Leu Thr Val Lys Lys Ile Phe Val Gly Gly Ile Lys Glu
   20 25 30
   Asp Thr Glu Glu Tyr Asn Leu Arg Asp Tyr Phe Glu Lys Cys Ser Lys
   35 40 45
   Ile Glu Thr Ile Glu Val Met Glu Asp Arg Gln Ser Gly Lys Lys Arg
   50 55 60
   Gly Phe Ala Phe Val Thr Phe Asp Asp His Asp Ile Ile Asp Lys Ile
   65 70 75 80
   Val Val Gln Lys Tyr His Thr Ile Asn Gly His Asn Cys Glu Val Lys
   85 90 95
   Lys Ala Leu Ser Lys Gln Glu Met Gln Ser Ala Gly Ser Gln Arg Gly
   100 105 110
   His Gly Gly Gly Ser Gly Asn Phe Met Gly Arg Gly Gly Asn Phe Gly
   115 120 125
   Gly Ala Gly Gly Asn Phe Gly Leu Gly Gly Asn Phe Gly Gly Arg Gly
   130 135 140
   Gly Tyr Gly Gly Gly Gly Gly Gly Ser Arg Gly Ser Tyr Gly Arg Gly
   145 150 155 160
   Asp Gly Gly Tyr Asn Arg Phe Gly Arg Asp Gly Gly Asn Tyr Gly Gly
   165 170 175
   Gly Pro Gly Tyr Ser Ser Arg Gly Gly Tyr Gly Gly Gly Gly Thr Gly
   180 185 190
   Tyr Gly Asn Gln Gly Gly Gly Tyr Gly Gly Gly Gly Gly Gly Tyr Asp
   195 200 205
   Gly Tyr Asn Glu Gly Gly Asn Phe Gly Gly Gly Asn Tyr Gly Gly Gly
   210 215 220
   Gly Asn Tyr Asn Asp Phe Gly Asn His Ser Gly Gln Gln Gln Ser Asn
   225 230 235 240
   Tyr Gly Pro Met Lys Gly Gly Ser Phe Gly Gly Arg Ser Ser Gly Ser
   245 250 255
   Pro Tyr Gly Gly Gly Tyr Gly Ser Gly Gly Gly Ser Gly Gly Tyr Gly
   260 265 270
   Ser Arg Arg Phe
   275